(19) Europäisches Patentamt
European Patent Office
Office européen des brevets

(11) Veröffentlichungsnummer: **0 406 992 B1**

# (12) EUROPÄISCHE PATENTSCHRIFT

(45) Veröffentlichungstag der Patentschrift: **15.03.95**

(51) Int. Cl.$^{6}$: **A61K 49/04**

(21) Anmeldenummer: **90250167.5**

(22) Anmeldetag: **29.06.90**

Die Akte enthält technische Angaben, die nach dem Eingang der Anmeldung eingereicht wurden und die nicht in dieser Patentschrift enthalten sind.

## (54) Neue, nicht-ionische Carboxamid-Kontrastmittel.

(30) Priorität: **05.07.89 US 375714**
**03.11.89 US 431527**

(43) Veröffentlichungstag der Anmeldung:
**09.01.91 Patentblatt 91/02**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 015 867**
**EP-A- 0 032 387**
**EP-A- 0 082 803**
**EP-A- 0 308 364**
**EP-A- 0 317 492**
**FR-A- 2 293 919**
**FR-A- 2 299 020**
**FR-A- 2 435 253**
**US-A- 4 021 481**

**CHEMICAL ABSTRACTS Band 108, Nr. 1, 4. Januar1988, Seite 518, Zusammenfassung Nr. 5483t, Columbus, Ohio, US; S. BRADA-MANTEet al.: "Nonionic x-ray contrast agents: detection of ambient temperaturei-somers of congested hexasubstituted ben-zenes with multipositional hinderedrota-tions" & Magn. Reson. Chem. 1987, Band25, Nr. 4, Seiten 283-292**

(73) Patentinhaber: **SCHERING AKTIENGESELL-SCHAFT**

**D-13342 Berlin (DE)**

(72) Erfinder: **Sovak, Milos, Dr.**
**5330 La Rescenta,**
**P.O. Box 2494**
**California 92067,**
**Rancho Santa Fe (US)**

**EP 0 406 992 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Technischer Bereich

Gegenstand der Erfindung sind Mittel zur Verwendung als nicht-ionische Kontrastmittel und deren Herstellung.

Hintergrund

Röntgenkontrastmittel zur Darstellung des Herz-Kreislaufsystems und von Körperhöhlen müssen niedrige Viskosität aufweisen, gut wasserlöslich und nichttoxisch sein und einen hohen Jodgehalt besitzen. Geringe Viskosität ist für eine schnelle Application, vorübergehenden Ersatz schnell fließenden Blutes, z.B. bei der Kardioangiographie oder hochdosierten Urographie, oder zur Kontrastverstärkung bei der Computertomographie unverzichtbar. Um nichttoxisch zu sein, muß das Mittel stark hydrophil, nicht-ionisch und von einer Osmolalität sein die nahe dem Körpermilieu liegt. Die bisher bekannten nichttoxischen monomeren Kontrastmittel besitzen zwar eine ausreichende biologische Verträglichkeit und brauchbare Viskosität, sind aber auf das Körpermilieu bezogen hyperosmolal. In der diagnostisch nutzbaren Konzentration von 300 mg I/ml überschreiten sie typischerweise den physiologischen Wert von 310 mOsm erheblich. Hyperosmolalität dieser Lösungen treibt zwangsläufig Wasser aus den Zellen, wodurch die Zeilmembranen zerstört, der Gesamtelektrolythaushalt gestört und die Auskleidung von Gefäßen oder Organhohlräumen geschädigt wird. Es hat sich auch gezeigt, daß Hyperosmolalität ein Grund für die Gefäßschmerzen ist, die beständig durch hyperosmolale Kontrastmittel hervorgerufen werden.

Die meisten nicht-ionischen Kontrastmittel, gleichgültig ob monomerer oder oligomerer Art, sind aromatische Amide mit einer oder mehreren an Stickstoff gebundenen Polyhydroxy-, niederen aliphatischen Alkyl- oder Acyl-Untergruppen. Der triiodierte Benzolring enthält eine Anzahl von räumlich eng miteinander verbundenen Funktionalitäten. Der Stand der Technik beschreibt verschiedene als Amide angelagerte Hydroxyalkylamine. Als Beispiele für diese Amine seien genannt: Serinol; 1-Amino-2,3-propandiol; N-Methyl-1-amino-2,3-propandiol; Aminotetritole, Ethanolamin, Diethanolamin oder Tromethamin.

Die Substitution der 1- und 3-ständigen Carboxylgruppen mit demselben Hydroxyalkylamin schränkt den Aufbau stark ein, da die praktisch verfügbaren Substituenten entweder zu klein sind, um das Molekül löslich zu machen, oder zu groß, um eine Lösung von niedriger Viskosität zu ergeben (Nycomed, US 4.021.481 und 3.701.771; Schering AG, U.S. 4.547.357, Bracco, U.S. 4.001.323). Aus diesem Grund wurden verbesserte Verbindungen entwickelt, in denen die beiden Hydroxyalkylamine verschieden waren (Schering AG U.S. 4.364.921). Derartige Verbindungen sind jedoch in der Synthese kompliziert und nur unter hohen Kosten darzustellen.

Einschlägige Veröffentlichungen

Die U.S. Patente Nr. 3.701.771, 4.001.323, 4.021.481, 4.364.921, 4.547.357 und die gleichzeitig anhängige U.S. Anmeldung Ser. Nr. 214,663, eingereicht am 1. Juli 1988, beschreiben verschiedene Verbindungen, von denen berichtet wird, daß sie für nicht-ionische Kontrastmittel nutzbar sind.

ZUSAMMENFASSUNG

Nicht-ionische Kontrastmittel auf der Grundlage eines asymmetrischen Triiodisophthaldiamids werden zur Verfügung gestellt, bei denen die verbleibende Ring-Position durch ein substituiertes Stickstoffatom besetzt ist, während eine der Carboxylgruppen ein nichtsubstituiertes Amid und die andere Carboxylgruppe mindestens ein mono-(Hydroxyalkyl)-substituiertes Amid ist. Das Molekül hat mindestens zwei Hydroxylgruppen.

BESCHREIBUNG SPEZIFISCHER AUSFÜHRUNGSFORMEN

Nicht-ionische Kontrastmittel auf der Grundlage eines Acylamid-substituierten Triiodisophthaldiamids werden zur Verfügung gestellt, wobei nur einer der Amid-Stickstoffe zumindest einfach substituiert ist. Die Verbindungen lassen sich kostengünstig mit guter Ausbeute herstellen und werden in hoher Reinheit erhalten. Die den Gegenstand der Erfindung bildenden Verbindungen erweisen sich als niedrig osmolal, während sie gleichzeitig mäßige bis niedrige Viskosität behalten.

Die erfindungsgemäßen Verbindungen entsprechen vorzugsweise der folgenden Formel (I) (mit Ausnahme des 3[N-(1,3-Dihydroxyprop-2-yl)carbamoyl]5-(2-Hydroxy-1-oxopropyl)amino-2,4,6-triiod-benzamids):

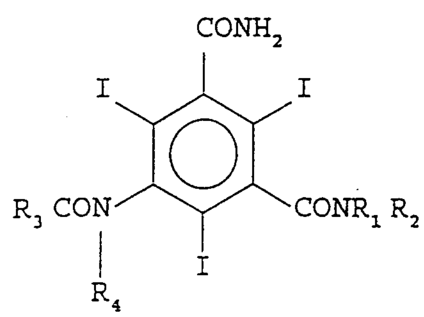

(I)

worin

$R_1$ Wasserstoff, ein niederes Alkyl oder Hydroxyalkyl bedeutet, wobei die Alkylgruppe zwischen 1-3, üblicherweise 1-2, Kohlenstoffatome aufweist, und die Hydroxyalkylgruppe mindestens 0,5 Hydroxyle pro Kohlenstoffatom und bis zu n-1 Hydroxylgruppen enthält, wobei n die Anzahl der Kohlenstoffatome bezeichnet, und das Hydroxyalkyl zwischen 2-6, üblicherweise 2-4, Kohlenstoffatome aufweist;

$R_2$ ist ein Mono- oder Polyhydroxyalkyl mit 2-6, üblicherweise 2-4, C-Atomen, welches mindestens eine Hydroxylgruppe und mindestens n-2 und höchstens n-1 Hydroxylgruppen aufweist, wobei n die Anzahl der Kohlenstoffatome ist;

$R_3$ ist ein niederes Alkyl, hydroxy-substituiertes niederes Alkyl oder niederes alkoxyl-substituiertes niederes Alkyl, wobei das Alkyl 1-4 C-Atome, üblicherweise 1-3 C-Atome, und das Alkoxyl 1-3, üblicherweise 1-2, C-Atome aufweisen und die Anzahl der Oxygruppen zwischen 0 und n-1, wobei n die Anzahl der C-Atome darstellt, variiert oder zwei $R_3$-Gruppen zusammengefaßt werden können, um eine Brücke von 0-2 C-Atomen, vorzugsweise 1 C-Atom, zu bilden; d.h. $R_3$ steht für

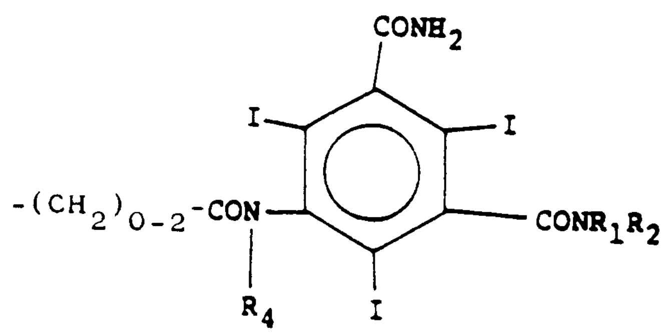

$R_4$ bedeutet Wasserstoff, ein niederes Alkyl oder Mono- oder Polyhydroxyalkyl, wobei die Alkylgruppen und die Hydroxylgruppen der vorstehend für analoge Gruppen beschriebenen Definition entsprechen.

Die Alkylgruppen können gerad- oder verzweigtkettig sein, üblicherweise geradkettig, wobei die C-Atome normalerweise nicht quaternär sind.

Der Alkyl-Rest in den Mono- oder Polyhydroxyalkylgruppen $R_1$ und $R_2$ weist üblicherweise 2-6 C-Atome, vorzugsweise 2-4 C-Atome auf. Die Gruppen haben vorzugsweise 1-5, üblicherweise 1-3 Hydroxygruppen. Diese Hydroxygruppen können primär, sekundär oder tertiär sein. Beispiele sind Tris-Hydroxymethylmethyl, Hydroxyethyl, Dihydroxypropyl und Trihydroxybutyl. Die Carbamide können unter Verwendung von 3-Amino-1,2-Propandiol, Serinol oder Amino-Tetritolen, d.h. Threitol und Erythritol entweder in D,L-Mischung oder in optisch reinen Formen, Ethanolamin oder Diethanolamin oder Tromethamin oder deren Derivaten hergestellt werden, wobei die Hydroxylgruppen in reversibler Form geschützt sind. $R_1$ bedeutet Wasserstoff oder ein niederes Alkyl, vorzugsweise Wasserstoff oder Methyl. $R_3$ ist ein niederes Alkyl oder Oxyalkyl mit 1-6, üblicherweise 1-4, C-Atomen, vorzugsweise Methyl, Hydroxymethyl oder Hydroxyethyl. Beispielshalber werden Alkoxylgruppen mit 1-3 C-Atomen, vorzugsweise 1-2 C-Atomen, enthaltende Alkoxyalkyle und Alkyle mit 1-3 C-Atomen, insbesondere Methoxymethyl, genannt. Alternativ werden zwei $R_3$-Gruppen zu einer Bindung, Methylen oder Ethylen, insbesondere Methylen, zusammengefaßt. $R_4$ bedeutet Wasserstoff, Alkyl oder Mono- oder Polyhydroxyalkyl mit 1-6, vorzugsweise 1-4, C-Atomen, einschließlich Methyl, Ethyl, Propyl, Hydroxyethyl und Dihydroxypropyl.

Zu den hier interessierenden monomeren Verbindungen gehören 5-[N-(2-Hydroxyethyl)-methoxyacetamid]-2,4,6-triiodo-3-[N-(1,3,4-trihydroxybut-2-yl)]carbamoyl-Benzamid; 5-[N-(2-Hydroxyethyl)-hydroxyacetamid]-2,4,6-triiodo-3-[N-(2,3-dihydroxypropyl)]carbamoyl-Benzamid; 5-[N-(2,3-Dihydroxypropyl)-acetamid]-2,4,6-triiodo-3-[N-(2,3-dihydroxypropyl)]carbamoyl-Benzamid; 5-[N-(2,3- Dihydroxypropyl)-glycolamido-2,4,6-triiodo-3-[N-(2-hydroxyethyl)]carbamoyl-Benzamid; 5-[N-(1,3,4-Trihydroxy-but-2-yl)-acetamid]-2,4,6-triiodo-3-[N-(2-hydroxyethyl)]carbamoyl-Benzamid; 5-[N-(Methyl)glycolamid]-2,4,6-triiodo-3-[N-(1,3,4-trihydroxy-erythro-but-2-yl]carbamoylbenzamid; und 5-[N-(Hydroxyethyl)acetamido]-2,4,6-triiodo-3-[N-(1,3,4-trihydroxythreobut-2-yl)]carbamoyl-Benzamid.

Zu den hier interessierenden dimeren Verbindungen gehören bis-[{3-N-(2,3-Dihydroxypropyl-carbamoyl)5-carbamoyl}-2,4,6-triiodo-N-(2,3-dihydroxypropyl)anilid-Malonsäure; bis-[{3-N-(2,3-Dihydroxypropyl-carbamoyl)5-carbamoyl}-2,4,6-triiodo-N-(2-hydroxyethyl)anilid-Malonsäure; und bis-[{3-N-(1,3,4-Trihydroxy-but-2-yl-carbamoyl)5-carbamoyl}-2,4,6-triiodo-N-methylanilid-Malonsäure.

Die den Gegenstand der Erfindung bildenden Mittel enthalten zwischen 50 und 52% Jod, üblicherweise etwa 51%, besitzen in einer Lösung von 300 mg I/ml bei 37 °C (cps) eine Viskosität im Bereich von 4 - 5 und eine Osmolalität in mOsm im Bereich von etwa 275 - 400, üblicherweise um 285 - 375, für 300 mg I/ml bei 37 °C in wässriger Lösung, während sie bei pharmazeutischen Zubereitungen zwischen ca. 300 - 400, üblicherweise zwischen 325 - 390 liegt.

Die erfindungsgemäßen Mittel werden unter konventionellen Bedingungen zubereitet. Üblicherweise werden die Zubereitungen ein wässriges Medium aufweisen, das ein physiologisch akzeptables chelatgebundenes Kalziumsalz enthält, z.B. EDTA, sowie einen Puffer zur Einstellung auf einen pH-Wert zwischen ca. 6,5 und 7,5, insbesondere etwa 7, bei dem es sich um Tris, Carbonat, Citrat oder Kombinationen derselben handeln kann. Als weitere Zusätze können Bicarbonat, Phosphat usw. verwendet werden. Das chelatgebundene Kalzium liegt im allgemeinen in einer Menge von 5 - 15, üblicherweise etwa 10 mg/100 ml vor, und der Puffer wird im allgemeinen in Mengen zwischen ungefähr 2 - 10 mM verwendet.

Die Erfindung betrifft ferner ein Verfahren zur Herstellung von Verbindungen der allgemeinen Formel I dadurch gekennzeichnet, daß man

a) Verbindungen der allgemeinen Formel V

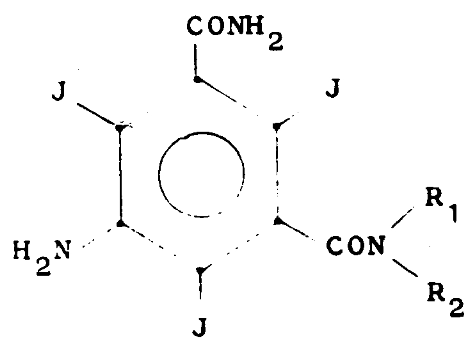

(V),

worin in $R_1$ und $R_2$ enthaltene Hydroxygruppen gegebenenfalls geschützt sind,

durch Umsetzung mit $R_3CO$-X, wobei X für Halogen oder einen Esterrest steht, in katalysierenden Lösungsmitteln wie z.B. Pyridin, DMA oder DMF und gegebenenfalls anschließender Abspaltung der Schutzgruppen in Endprodukte der allgemeinen Formel I mit $R_4$ in der Bedeutung von Wasserstoff oder in Zwischenprodukte der allgemeinen Formel I mit $R_4$ in der Bedeutung von $R_3CO$ überführt und diese, gegebenenfalls mit geschützten Hydroxygruppen, anschließend gewünschtenfalls durch Alkylierung unter basischen Bedingungen mit $R_4$-enthaltenden Reagenzien, gegebenenfalls nach folgender Abspaltung der Schutzgruppen, in Endprodukte der allgemeinen Formel I, in welcher $R_4$ nicht Wasserstoff bedeutet, umwandelt bzw.

b) Verbindungen der allgemeinen Formel VI

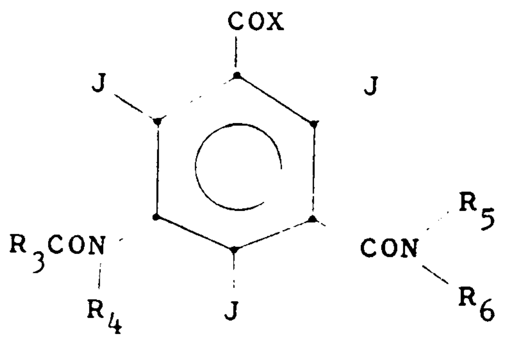

(VI)

mit $R_5$ in der Bedeutung von $R_1$ oder Wasserstoff, $R_6$ in der Bedeutung von $R_2$ oder Wasserstoff und X in der Bedeutung von Halogen oder eines Esterrestes,
mit Ammoniak bzw. mit Reste $R_1$ und $R_2$ enthaltenen Hydroxyalkylaminen, deren Hydroxygruppen geschützt sein können, umsetzt und anschließend gegebenenfalls die Schutzgruppen abspaltet.

Zur Herstellung der erfindungsgemäßen Verbindungen können herkömmliche Reaktionen zu einem festgelegten Reaktionsweg kombiniert werden. Nach einem erfindungsgemäßen Verfahren können die erfindungsgemäßen Produkte beispielsweise aus der Verbindung der Formel (II) hergestellt werden:

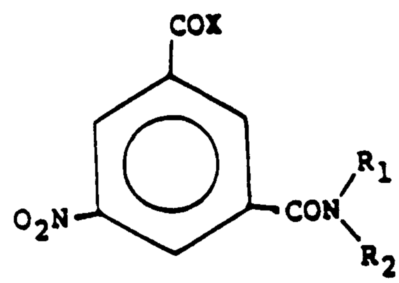

II

worin
$R_1$ und $R_2$ die obenangegebene Bedeutung haben und X entweder einen niederen Alkylester oder ein Halogen bedeutet.

Verbindungen der allgemeinen Formel (II) werden dargestellt, indem man ein Hydroxyalkylamin ($NHR_1,R_2$), dessen OH-Gruppen geschützt oder ungeschützt sein können, mit einem handelsüblichen Monoester der 5-Nitroisophthalsäure reagieren läßt und anschließend die verbleibende Carboxylgruppe aktiviert. Diese Aktivierung wird in geeigneter Weise erreicht, wenn X ein Halogen wie Cl, Br, I, oder ein Alkoxy ist, wobei Chlor und Methoxy bevorzugt sind.

Die Estergruppe des 5-Nitroisophthalsäure-Monoesters wird mit dem Hydroxyalkylamin der allgemeinen Formel $HNR_1R_2$ gemäß obiger Definition aminolysiert, oder die Aminolysierung kann zunächst mit Ammoniak erreicht werden. Die verbleibende Carboxylgruppe wird dann wie oben beschrieben aktiviert. Wenn die Hydroxylgruppen des $NR_1R_2$-Rests ungeschützt sind und durch die Aktivierung beeinträchtigt werden können, können sie in herkömmlicher Weise, z.B. O-Acetylierung oder Isopropylidierung, in geeigneter Weise geschützt werden.

Die Verbindung der allgemeinen Formel (II) kann in herkömmlicher Weise aus Wasser oder niederen Alkoholen kristallisiert werden.

Nach Reaktion mit wasserfreiem Ammoniak oder Ammoniumhydroxid, wobei das asymmetrische Isophthaldiamid anfällt, wird die Verbindung in herkömmlicher Weise hydrogeniert, triiodiert und acyliert, worauf man zur allgemeinen Formel (III) gelangt:

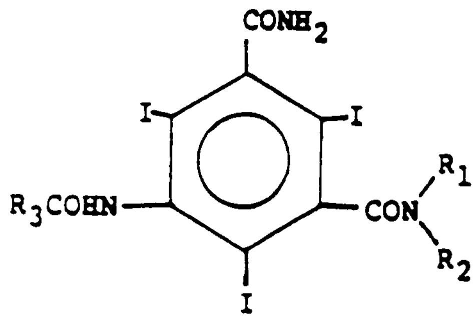

III

worin

$R_1$, $R_2$ und $R_3$ die oben angegebene Bedeutung haben. Reduktion und Iodierung erfolgen in bekannter Weise nach Standardverfahren unter Verwendung eines Katalysators, Palladium auf Kohle oder Raney-Nickel in Wasser oder niederen Alkoholen, und von Wasserstoff unter niedrigem oder hohem Druck. Das entstehende 5-Aminoisophthalsäure-Diamid wird dann in bekannter Weise iodiert. Die Verbindung erhält man dann in einfacher Weise durch Kristallisation aus dem Reaktionsgemisch, man wäscht, trocknet und acyliert sie nach ebenfalls bekannten Verfahren. So können ein aktiviertes Acyl $R_3CO$-X mit X als Halogen oder der gleichen zur Anhydridbildung verwendeten Acylgruppe, und katalysierende Lösungsmittel wie Pyridin, DMA oder DMF verwendet werden.

Wenn Verbindungen nach Formel (I), wobei $R_4$ kein Wasserstoff ist, hergestellt werden, erfolgt die Alkylierung nach üblichen Verfahren. Die Reaktion mit Hydroxyalkylresten erfolgt, ausgenommen für die niederen Alkyle, die zweckmäßigerweise in einem früheren Syntheseschritt eingeführt werden, typischerweise im letzten Schritt. Die Alkylierung kann in einem Glykolsolvens mit hohem Siedepunkt, z.B. Ethylen- oder Propylenglykol, ausgeführt werden; einen pH-Wert im hochbasischen Bereich erzielt man mit Natriummethoxid, Natriumhydroxid oder anderen anorganischen oder organischen Basen.

Andererseits ist es, wenn Nebenprodukte entstehen, die ansonsten mit herkömmlichen Reinigungsverfahren nur schwer zu entfernen wären, manchmal vorteilhaft, zum Zwecke der Reinigung die Anwesenheit einer Carboxylgruppe bis zu den letzten Stufen der Reaktionsfolge aufrechtzuerhalten, damit die Verbindung leicht als Salz in Wasser gelöst und anschließend mit einer anorganischen Säure ausgefällt oder nochmals ausgefällt werden kann. Zu den bevorzugten Salzen gehören Ammonium, Natrium, Kalium, Kalzium, Barium oder Lithium.

Dieses alternative Verfahren beruht auf der vorstehend beschriebenen Reduktion, Iodierung und Acetylierung von Verbindungen der Formel (II) (in denen X = OH ist), womit man zu der Formel (IV) gelangt:

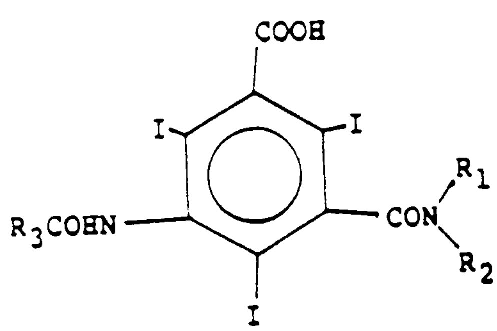

IV

worin

$R_1$, $R_2$ und $R_3$ die oben angegebene Bedeutung haben.

Die Hydroxylgruppen werden vor Aktivierung der Carboxylgruppe geschützt. Außerdem muß das Anilid mit $R_3CO$ diacyliert werden, da dieser Stickstoff bei der Carboxylaktivierung ebenfalls beeinträchtigt würde. Die Carboxylgruppe wird wie oben beschrieben, vorzugsweise mit Säurechlorid, jedoch können auch gemischte Anhydride wie t-Butyloxycarbonyl verwendet werden, aktiviert. Verbindungen nach Formel (IV) lassen sich leicht aus einem aprotischen Lösungsmittel kristallisieren, worauf man sie mit Ammoniumhydroxid oder Ammoniak reagieren läßt und als letzten Schritt einer Alkylierung unterzieht.

Eine wirksame Amidierung erfordert normalerweise, daß entweder ein Überschuß einer amidierende Base als Säureakzeptor oder alternativ tertiäre Amine, wie Triethylamin, Tributylamin, Pyridin, oder anorganische Basen, wie Bicarbonat oder Carbonat verwendet werden.

Eine weitere Variante des Syntheseverfahrens läßt sich anwenden, wenn unerwünschte Nebenprodukte auftreten. So kann die Alkylierung des Anilids vor der Amidierung stattfinden. Die Alkylierung der Verbindungen der allgemeinen Formel (IV), in der $R_1$, $R_2$ und $R_3$ die oben angegebene Bedeutung haben, kann wie oben beschrieben nach bekannten Verfahren durchgeführt werden.

Bei der Alkylierung zur Einführung von $R_4$ gehen die Schutzgruppen $R_3CO$ verloren, und daher müssen die Hydroxylgruppen von $R_1$ und $R_2$ (wenn vorhanden auch $R_4$) vor der Carboxyl-Aktivierung und anschließenden Amidierung mit Ammoniak neu geschützt werden. Hierfür werden meistens Acetylgruppen verwendet.

Nach dem letzten Schritt können die Schutzgruppen, wenn die Hydroxylgruppen noch geschützt sind, was der Fall ist, wenn die Alkylierung nicht den letzten Schritt bildet, leicht mit Standardmitteln entfernt werden, z.B. durch Behandlung mit Ionenaustauscherharzen oder Verwendung von Säuren oder Basen in katalytischen Mengen in alkoholischen oder wässrigen Lösungsmitteln.

Nach bekannten Verfahren kann man eine Entsalzung erreichen. Typischerweise werden die Salze mittels Ionenaustauschern entweder im Mischbettverfahren oder in getrennten Säulen die jeweils ein nicht-ionisches bzw. kationisches Austauscherharz enthalten, entfernt. Wahlweise können die Verbindungen der allgemeinen Formel (I) auch an einem polystyrolabsorbierenden Neutralharz absorbiert und danach eluiert werden.

Nach Entfernung der Salze kann das Produkt nun aus einer Vielzahl von Lösungsmitteln, vorzugsweise niederen Alkoholen, kristallisiert werden. Entfärbung erreicht man durch Rückflußkochen in wäßriger Lösung über Aktivkohle.

Das Dimer wird zweckmäßigerweise aus dem Benzamid dargestellt, wobei der Aminostickstoff alkyliert und die verbleibende Carbonylgruppe, z.B. als Chlorcarbonyl, aktiviert wird. Die zweibasige Säure wird in der aktivierten Form, insbesondere als Diacyldichlorid in einem organischen, aprotischen polaren Lösungsmittel, verwendet. Das an den Ringkohlenstoff gebundene Chlorcarbonyl wird dann zum Endprodukt hydroxyalkylamidiert.

Die erfindungsgemäßen Verbindungen und die Verbindungen nach der allgemeinen Formel (I) sind in wässriger Lösung stabil; sie bilden bereitwillig übersättigte Lösungen, die ebenfalls stabil sind. Die Lösungen können mit üblichen Mitteln im Autoklaven behandelt werden. Bei diagnostisch nutzbaren Jodkonzentrationen weisen die Verbindungen Osmolalitäten auf, die typischerweise sehr nahe bei den physiologischen Werten liegen. Gleichzeitig ist die Viskosität der Lösung gering. Das Ziel der Erfindung, nämlich die allgemein bekannte Tatsache der sich gegenseitig ausschließenden Forderungen nach einer niedrigen Osmolalität und niedrigen Viskosität zu überwinden, ist damit erreicht. Die neuartigen Verbindungen besitzen infolgedessen eine ausgezeichnete lokale und systemische Verträglichkeit. Die Verbindungen werden biologisch gut toleriert und haben einen hohen Jodgehalt, insbesondere im Vergleich mit den derzeit verfügbaren nicht-ionischen Röntgenkontrastmitteln.

Als Beispiele für die Eigenschaften der Verbindungen aus der Klasse der Benzamide wurden Daten für die Verbindungen (11) (Anspruch 5) und (19) (Anspruch 6) zusammengestellt. Tabelle I zeigt zum Vergleich außerdem Daten für Verbindungen des bisherigen Stands der Technik.

TABELLE 1

GRUNDEIGENSCHAFTEN VON NICHT-IONISCHEN MONOMEREN RÖNTGENKONTRASTMITTELN

| | Jod in % des Molekulargewichts | Viskosität von 300 mg I/ml bei 37 °C (cps) | Osmolalität von 300 mg I/ml Lösungen | | Näherungsbereich i.v. $LD_{50}$ g I/kg | |
|---|---|---|---|---|---|---|
| | | | wäßrige Lösung (mOsm) | pharmazeutisch formul. Lösung (mOsm) | männl. Maus 16-20 g | männl. Ratte 80-100 g |
| Iohexol | 46 | 6,3* | 650 | 685 | 16-17 | 11,5-12,5 |
| Iopamidol | 49 | 4,7* | 671 | 704 | 16-18 | 12-13 |
| Iopromid | 48 | 4,6* | 607 | 630 | 14-16 | 11-12 |
| Ioxilan | 48 | 4,7 | 560 | 588 | 16-18 | 12-13 |
| Verbindung 19 | 51 | 4,1 | 365 | 390 | 14-16 | 13-14 |
| Verbindung 11 | 51 | 4,2 | 300 | 326 | 15,5-16,5 | 15,5-17,5 |
| Verbindung 41 | 52 | 3,5 | 314 | 322 | 16-17,5 | 15-17 |

* Laut Literaturangabe; die anderen Daten wurden in unserem Labor ermittelt.
Alle Lösungen wurden, soweit nichts anderes angegeben ist, pharmazeutisch formuliert.

Die beobachtete niedrige Osmolalität und gleichzeitige geringe Toxizität wurde bisher nur bei nicht-ionischen Dimeren erreicht, die aber wegen ihrer hohen Viskosität, die bei vergleichbarer Lösungskonzentration mindestens beim Zweifachen liegt, in der allgemeinen Uroangiographie nicht verwendet werden können.

Die neuartigen Verbindungen besitzen eine ausgezeichnete biologische Verträglichkeit, da hohe Osmolalität

der ursächliche Faktor von Gefäßschmerzen und erheblichen Nebeneffekten bei der Darstellung der Periphergefäße der Gliedmaßen ist. (Sovak, M., Current Contrast Media and Ioxilan, Comparative evaluation of Vascular Pain by Aversion Conditioning, Investigative Radiology September 1988 Supp). Dies ist eine der bedeutenden diagnostischen Methoden der Gefäßröntgenologie. Es wird erwartet, daß die erfindungsgemäßen neuartigen Verbindungen bei diesen Methoden praktisch schmerzfrei angewendet werden können. Wegen ihrer physiochemischen und pharmakologischen Eigenschaften eignen sich die neuen Verbindungen als wasserlösliche Kontrastmittel für die Darstellung der Harnwege und des Herz-Kreislaufsystems, für Körperhöhlen und zur allgemeinen Kontrastverstärkung in der Computertomographie. Die Injektionslösungen der neuen Verbindungen lassen sich durch Auflösen in Wasser und Zugabe von üblichen physiologisch kompatiblen Puffern und Stabilisatoren, wie z.B. Komplexbildner, herstellen. Bei Formulierung mit Trägerstoffen, wie sie in den Pharmakopöen üblicherweise verwendet werden, sind die Verbindungen auch für enterale Anwendung geeignet. Die Dimere finden besonders in der Myelographie Anwendung.

Zur intravaskulären Verwendung enthalten die erfindungsgemäßen Verbindungen 20 - 80 Gew./Vol.-%, wobei Jodkonzentrationen von 150 - 400 mg/ml bevorzugt werden.

Die folgenden Beispiele dienen lediglich der Erläuterung und schränken den Erfindungsumfang nicht ein.

EXPERIMENTELLER TEIL

**BEISPIEL 1**

Amidierung von 5-Nitroisophthalsäure-Monomethylester (1) mit (Threo)-2-amino-1,3,4-butantriol zu: 5-Nitro-3{N-(1,3,4-trihydroxy-threo-but-2-yl)}carbamoyl-Benzoesäure (2)

Die Ausgangssubstanz (1, 22,5 g, 0,1 mol) wurde mit (Threo)-2-Amino-1,3,4-butantriol (30,25 g, 0,25 mol) gemischt und die Suspension wurde 30 Minuten lang auf 110-120 °C erwärmt. Die DSC zeigte vollständige Umsetzung in das Produkt, und die Lösung wurde in 1 n Salzsäure (200 ml) gegeben, um das Produkt auszufällen. Nach Abkühlen über Nacht wurde das Produkt filtriert und mit Eiswasser gewaschen (20 ml x 2). Trockung im Vakuum ergab eine weiße Festsubstanz (2, 21,0 g, 67% Ausbeute).

**BEISPIEL 2**

Veresterung von 5-Nitro-3-{N-(1,3,4-trihydroxy-threo-but-2-yl)}carbamoyl-Benzoesäure (2) mit Dimethylsulfat zu: 5-Nitro-3-{N-(1,3,4-trihydroxy-threo-but-2-yl)}carbamoyl-Methylbenzoat (3)

Die Titelverbindung (2, 15,7 g, 0,05 mol) wurde in 1 n Natriumhydroxidlösung (55 ml) gelöst und die Lösung auf < 20 °C gekühlt. Während 5 Minuten wurde Dimethylsulfat (9,45 g, 0,075 mol) zugegeben, und durch gelegentliche Zugabe von 5 n Natriumhydroxidlösung wurde der pH-Wert bei 8-10 gehalten. Die Lösung wurde 12 h bei Raumtemperatur gerührt, danach wurde der unlösliche Feststoff abfiltriert. Die pastöse Festsubstanz wurde mit kaltem Wasser (50 ml x 2) gewaschen und im Vakuum zu einem Pulver getrocknet (3, 11,8 g, 72 % Ausbeute).

**BEISPIEL 3**

Amidierung von 5-Nitro-3-{N-(1,3,4-trihydroxy-threo-but-2-yl)}carbomoyl-Methylbenzoat (3) mit Ammoniak zu: 5-Nitro-3-{N-(1,3,4-trihydroxy-threo-but-2-yl)carbamoyl-Benzamid (4)

Der Ester (3, 10,0 g, 0,0305 mol) wurde in Methanol (50 ml) gelöst und mit konzentriertem Ammoniumhydroxid (20 ml, ca. 0,3 mol) versetzt. Die Suspension wurde in einem geschlossenen Gefäß für 30 Minuten auf 50 - 60 °C erwärmt; die anschließende DSC zeigte den Abschluß der Reaktion an. Methanol und Ammoniumhydroxid wurden durch Destillation entfernt und durch $H_2O$ (50 ml) ersetzt. Man ließ die Mischung über Nacht abkühlen, anschließend wurde das unlösliche Produkt abfiltriert und mit kaltem Wasser (5 ml x 2) gewaschen. Vakuumtrocknung ergab das weiße, gemischte Amid (4, 7,15 g, 75 % Ausbeute).

**BEISPIEL 4**

Chlorierung von 5-Nitroisophthalsäure-Monomethylester (1) mit Thionylchlorid zu: 5-Nitroisophthalsäure-Monomethylester-Monosäurechlorid (5)

Die Titelverbindung (1, 225 g, 1 mol) wurde in Ethylacetat (0,5 l) gelöst und mit N,N-Dimethylformamid (0,1 ml) als Katalysator versetzt. Die Lösung wurde auf 70 °C erwärmt und mit Thionylchlorid (219 ml, 3 mol) innerhalb von 1,25 h versetzt. Die Temperatur wurde anschließend 2 Stunden lang auf 70 °C gehalten.

Das Thionylchlorid wurde zusammen mit Ethylacetat (200 ml x 3) abdestilliert und das Produkt in heißem Ethylacetat (250 ml) gelöst und mit Cyclohexan (1 l) ausgefällt, filtriert und mit Cyclohexan (200 ml x 2) gewaschen. Trocknung im Vakuum bei 50 °C ergab eine weiße Festsubstanz (5, 216 g, 89 % Ausbeute).

**BEISPIEL 5**

Amidierung von 5-Nitroisophthalsäure-Monomethylester-Monosäurechlorid (5) mit Aminodioxepan zu: 5-Nitro-3-{N-(trans-2,2-dimethyl-6-hydroxy-1,3-dioxepan-5-yl)}carbamoyl-Methylbenzoat (6)

Das Monoester-Monochlorid (5, 100 g, 0,411 mol) wurde in trockenem Tetrahydrofuran (1 l) gelöst und über 15 Minuten mit festem Aminodioxepan (132,8 g, 0,825 mol) portionsweise versetzt, wobei die Temperatur mit Hilfe eines Eisbades unter 25 °C gehalten wurde. Anschließend wurde die heterogene Mischung 30 Minuten lang bei Raumtemperatur gerührt, woraufhin die DSC das Ende der Reaktion anzeigte.

Das unlösliche Aminohydrochlorid wurde abfiitriert und das Tetrahydrofuran durch Destillation aus dem Filtrat entfernt. Der Rückstand wurde in Ethylacetat (400 ml) nahe am Siedepunkt gelöst; die Lösung ließ man mehrere Tage stehen, bis die Kristallisation des Produkts abgeschlossen war. Die Festsubstanz wurde abfiltriert, mit kaltem Ethylacetat (50 ml x 2) gewaschen und im Vakuumofen getrocknet; man erhielt ein weißliches Produkt (6, 82,4 g, 55 % Ausbeute).

**BEISPIEL 6**

Amidierung von 5-Nitro-3-{N-(trans-2,2-dimethyl-6-hydroxy-1,3-dioxepan-5-yl)}carbamoyl-Methylbenzoat (6) mit Ammoniumhydroxid zu: 5-Nitro-3-{N-(trans-2,2-dimethyl-6-hydroxy-1,3-dioxepan-5-yl)}carbamoyl-Benzamid (7)

Ein Parr-Druckreaktor (800 ml) wurde mit der Titelverbindung (6, 80 g, 0,22 mol), Methanol (110 ml) und 15 n Ammoniumhydroxid (225 ml, 3,38 mol) beschickt. Das Reaktionsgefäß wurde geschlossen und 2 Stunden lang in einem Wasserbad bei 50 °C untergetaucht; die anschließende DSC zeigte vollständige Umsetzung. Das heterogene Reaktionsgemisch wurde mit Wasser (100 ml) gemischt und dann als Schaum gestrippt. Der Schaum wurde in Wasser (100 ml) aufgeschlämmt, filtriert und zweimal mit 50 ml $H_2O$ gewaschen; man erhielt eine weiße Festsubstanz (7, 60,4 g, 79 % Ausbeute).

**BEISPIEL 7**

Reduktion und Spaltung von 5-Nitro-3-{N-(trans-2,2-dimethyl-6-hydroxy-1,3-dioxepan-5-yl)}-carbamoyl-Benzamid (7) mit Wasserstoff und Palladium auf Kohle und Salzsäure zu: 5-Amino-(hydrochlorid)-3-{N-(1,3,4-trihydroxy-threo-but-2-yl)}carbamoyl-Benzamid (8)

Ein Parr-Druckreaktionsapparat (2,0 l) wurde mit der Titelverbindung (7, 58 g, 0,16 mol), 1 n Salzsäure (410 ml) und Palladium auf Kohle (10 % Pd/C, 5,8 g, 1 % Gew./Gew. Pd) beschickt. Das Reaktionsgefäß wurde an ein Hydriergerät angeschlossen und 2 h unter Wasserstoffgas bei 50 psi (3,5 kp/cm$^2$) geschüttelt; die anschließende HPLC zeigte 90%ige Umsetzung in das Produkt (8). Der Palladiumkatalysator wurde abfiltriert, und das bei der Entschützung gebildete Aceton wurde im Vakuum bei 50 °C entfernt. Die entstandene klare Lösung (8, 450 ml, 90% Ausbeute) wurde direkt zur Iodierung verwendet.

**BEISPIEL 8**

Iodierung von 5-Amino-(hydrochlorid)-3-{N-(1,3,4-trihydroxy-threo-but-2-yl)}carbamoyl-Benzamid (8) mit Iodmonochlorid zu: 5-Amino-2,4,6-triiod-3-{N-(1,3,4-trihydroxy-threo-but-2-yl)}carbamoyl-Benzamid (9)

Die Titelverbindung (8, 0,14 mol) in 1 n Salzsäure (450 ml) wurde auf 85 °C erwärmt und mit Iodmonochlorid (135 ml, 0,49 mol) versetzt. Das Reaktionsgemisch wurde für 2 Stunden auf 85 °C erwärmt; die anschließende HPLC zeigte den Abschluß der Reaktion an. Das Reaktionsgemisch wurde auf 25 °C gekühlt und mit 2 x Cyclohexen (200 ml), 3 x Dichlormethan (300 ml) und 5 x Chloroform (200 ml) extrahiert, bis die purpurne Farbe der wässrigen Phase verschwunden war. Die entstandene hellgelbe Lösung wurde in einer Säule mit Duolite-Harz A340 (800 g) und Dowex-Harz 50W-X8 (266 g) rezirkuliert. Die Harze wurden mit $H_2O$ (6 l) gespült und die Lösung auf 300 ml eingeengt, woraufhin eine weiße, kristalline Festsubstanz auszukristallisieren begann. Nach Filtration des Produkts erhielt man eine weiße Festsubstanz (9, 40 g, 0,06 mol, 43 % Ausbeute).

**BEISPIEL 9**

Acetylierung von 5-Amino-2,4,6-triiod-3-{N-(1,3,4-trihydroxy-threo-but-2-yl)}carbamoyl-Benzamid (9) mit Essigsäureanhydrid zu: 5-Diacetylamino-2,4,6-triiod-3-{N-(1,3,4-triacetoxy-threo-but-2-yl)}carbamoyl-Benzamid (10)

Die Titelverbindung (9, 90 g, 0,14 mol) wurde mit Essigsäureanhydrid (500 ml, 4,95 mol) bei 70 °C unter starkem Rühren gemischt. Als Katalysator wurde Perchlorsäure (0,36 ml, 0,004 mol) zugegeben, wodurch die Temperatur auf 85 °C stieg. Das Reaktionsgemisch wurde 1 h bei 85 °C gerührt; es wurde dann homogen und die DSC zeigte den Abschluß der Reaktion an. Zur Neutralisierung der Perchlorsäure wurde Natriumacetat (0,33 g, 0,004 mol) zugesetzt, das Lösungsmittel wurde entfernt, und man erhielt ein zähflüssiges, braunes Öl. Das Öl wurde bei 70 °C mit Butylacetat (200 ml) verdünnt und das Lösungsmittel anschließend entfernt. Der Strippvorgang wurde zweimal wiederholt; man erhielt einen braunen Schaum (10, 113 g, 0,13 mol, 93 % Ausbeute).

**BEISPIEL 10**

Deacetylierung und Alkylierung von 5-Diacetylamino-2,4,6-triiod-3{N-(1,3,4-triacetoxy-threo-but-2-yl)-}carbamoyl-Benzamid (10) mit Natriummethoxid und 2-Chlorethanol zu: 5-{N-(2-Hydroxyethylacetamido)}-2,4,6-triiod-3-{N-(1,3,4-trihydroxy-threo-but-2-yl)}carbamoyl-Benzamid (11)

Die Titelverbindung (10, 113 g, 0,13 mol) wurde in Methanol (500 ml) gelöst, dem bei 50 °C 25%-iges Natriummethoxid (55 g, 0,25 mol) zugegeben wurden. Nach 5 h zeigte die HPLC den Abschluß der Deacetylierung an, und die Lösung wurde mit Dowex-Harz 50 W-X4 (10 g) neutralisiert. Das Harz wurde abfiltriert und das Filtrat auf 400 ml eingeengt. Die neutrale methanolische Lösung wurde auf 45 °C erwärmt und mit Trinatriumphosphatdodecahydrat (129 g, 0,34 mol) und 2-Chlorethanol (18,2 ml, 0,272 mol) versetzt. Das Reaktionsgemisch wurde 48 h bei 45 °C gerührt, dann gab man Chlorethanol (4,7 ml, 0,07 mol) und Natriummethoxid (14,7 g, 0,07 mol) hinzu. Nach 71 h zeigte die HPLC den Abschluß der Reaktion an. Die unlöslischen Salze (89 g) wurden durch Filtrieren entfernt und die Lösung mit Salzsäure (6 N, 7 ml) neutralisiert. Nach Einengen der Lösung erhielt man einen braunen Schaum (11) (94 g, 0,12 mol, 92 % Ausbeute).

**BEISPIEL 11**

Amidierung von 5-Nitroisophthalsäure-Monomethylester (1) mit 3-Amino-1,2-propandiol zu: 5-Nitro-3-{N-(2,3-dihydroxypropyl)}carbamoyl-Benzoesäure (12)

Die Ausgangssubstanz (1, 225 g, 1 mol) wurde mit 3 Amino-1,2-propandiol (227,8 g, 2,5 mol) gemischt, und das heterogene Gemisch wurde 1 h auf 110-120 °C erhitzt. Damit war die Reaktion abgeschlossen, und das homogene Gemisch wurde mit Wasser (1 l) und konz. HCl (170 ml) gemischt. Das Gemisch wurde mehrere Tage lang gekühlt, um das Produkt vollständig auszufällen, die Festsubstanz wurde abfiltriert und mit kaltem Wasser (50 ml x 2) gewaschen. Nach Vakuumtrocknung erhielt man eine weiße Festsubstanz (12, 193 g, 68 % Ausbeute).

**BEISPIEL 12**

Reduktion von 5-Nitro-3-{N-(2,3-dihydroxypropyl)} carbamoyl-Benzoesäure (12) mit Wasserstoff und Palladium auf Kohle zu: 5-Amino-(hydrochlorid)-3-{N-(2,3-dihydroxypropyl)}-carbamoyl-Benzoesäure (13)

Die Nitrosäure (12, 180 g, 0,634 mol) wurde mit Wasser (1 l) gemischt und mit konzentrierter HCl (60 ml) und 10% Palladium auf Kohle (18 g) versetzt. Die Suspension wurde bei 2-4 atm. bis zur Druckkonstanz hydriert, zu diesem Zeitpunkt zeigten HPLC und DSC das Reaktionsende an. Der Palladium-Katalysator wurde durch Filtrieren entfernt und die homogene Lösung wurde, ohne das Produkt zu isolieren, für die folgende Umsetzung verwendet (13, ungefähre Ausbeute 98 %).

**BEISPIEL 13**

Iodierung von 5-Amino-(hydrochlorid)-3-{N-(2,3-dihydroxypropyl)}carbamoyl-Benzoesäure (13) mit Iodmonochlorid zu: 5-Amino-2,4,6-triiod-3-{N-(2,3-dihydroxypropyl)}-carbamoyl-Benzoesäure (14)

Die Titelverbindung (13, ca. 0,62 mol in 1,5 l Wasser) wurde mit Wasser weiter auf ein Gesamtvolumen von 4 l verdünnt und auf 85 °C erhitzt. Während 20 Minuten wurde Iodmonochlorid (4,1 molar, 499 ml, 2,05 mol) zugegeben, und die Temperatur wurde 6-8 h bei 90 °C gehalten. Das Ende der Umsetzung wurde durch HPLC nachgewiesen. Das homogene Gemisch wurde gekühlt, mit 1,2-Dichlorethan:Cyclohexen (9:1, 500 ml x 2) und anschließend mit 1,2-Dichlorethan (250 ml x 2) extrahiert. Die wässrige Phase wurde dann durch Destillation auf 0,9 l eingeengt und mehrere Tage lang bis zur vollständigen Abscheidung des Feststoffes gekühlt. Nach Filtrierung, Waschen mit kaltem Wasser (100 ml x 2) und Vakuumtrocknung erhielt man das gelbbraune Produkt (14, 286 g, 73 % Ausbeute).

**BEISPIEL 14**

Acetylierung von 5-Amino-2,4,6-triiod-3-{N-(2,3-dihydroxypropyl)}carbamoyl-Benzoesäure (14) mit Essigsäureanhydrid zu: 5-Diacetylamino-2,4,6-triiod-3-{N-(2,3-diacetoxypropyl)}carbamoyl-Benzoesäure (15)

Die Ausgangssubstanz (14, 100 g, 0,158 mol) wurde mit Essigsäureanhydrid (300 ml, 3,16 mol) und 70%-iger Perchlorsäure (0,2 ml) gemischt und 8 h auf 80-90 °C erhitzt. Das Gemisch wurde mit wasserfreiem Natriumacetat (0,25 g) neutralisiert, und das Essigsäureanhydrid und die Essigsäure wurden durch Destillation bei 70-80 °C entfernt. Der ölige Rückstand wurde mit Butylacetat (100 ml x 2) azeotrop destilliert, dann in Ethylacetat (250 ml) gelöst und direkt für die Chlorierung verwendet (15, ungefähre Ausbeute 90 %).

**BEISPIEL 15**

Chlorierung von 5-Diacetylamino-2,4,6-triiod-3-{N-(2,3,-diacetoxypropyl)}carbamoyl-Benzoesäure (15) mit Thionylchlorid zu: 5-Diacetylamino-2,4,6-triiod-3-{N-(2,3-diacetoxypropyl)}carbamoyl-Benzoylchlorid (16)

Die Ausgangssubstanz (15, ca. 0,142 mol) in Ethylacetat (225 ml) wurde bei 65-70 °C mit Thionylchlorid (57 ml, 0,78 mol) versetzt; anschließend wurde die Temperatur für 1 h auf 75-80 °C erhöht. Thionylchlorid und Ethylacetat wurden unter Vakuum abdestilliert. Der Rückstand wurde mit Butylacetat (100 ml x 2) azeotrop destilliert und vakuumgetrocknet. Der braune Schaum (16, ca. 130 g, geschätzte Ausbeute 95 %) wurde direkt in den anschließenden Amidierungsschritt übernommen.

**BEISPIEL 16**

Amidierung von 5-Diacetylamino-2,4,6-triiod-3-{N-(2,3-diacetoxypropyl)}carbamoyl-Benzoylchlorid (16) mit Ammoniak zu: 5-Acetylamino-2,4,6-triiod-3-{N-(2,3-diacetoxypropyl)}carbamoyl-Benzamid (17)

Das Säurechlorid (16 ca. 0,135 mol) wurde in trockenem N,N-Dimethylacetamid (15 ml) gelöst. Diese Lösung wurde auf 0-5 °C gekühlt, unter Verwendung eines Trockeneis/Aceton -Kühlers wurde wasserfreies Ammoniak (ca. 20 ml) in das Gemisch kondensiert, und das Reaktionsgemisch wurde 24 h bei Raumtemperatur unter Verschluß gehalten. Ammoniak und DMA wurden durch Vakuumdestillation entfernt. Mit 1-Pentanol (500 ml) wurde eine Festsubstanz abgeschieden, die abfiltriert und mit 1-Pentanol (150 ml x 2)

gewaschen wurde. Nach Trocknen im Vakuum erhielt man eine gelbbraune Festsubstanz (17, 82 g, 80,2 % Ausbeute).

**BEISPIEL 17**

Deacetylierung von 5-Acetylamino-2,4,6-triiod-3-{N-(2,3-diacetoxypropyl)}carbamoyl-Benzamid (17) zu: 5-Acetylamino-2,4,6-triiod-3-{N-(2,3-dihdroxypropyl)}carbamoyl-Benzamid (18)

Die Titelverbindung (17, 81,2 g, 0,107 mol) wurde in Wasser (203 ml) suspendiert und dann durch Zutropfen von 50 % Gew./Gew. Natriumhydroxid in Wasser (16,9 ml, 0,322 mol) behandelt. Unter Rühren wurde eine vollständige Lösung erreicht. Die Lösung wurde 30 Minuten lang im Vakuum entgast, woraufhin 12 M HCl (15 ml, 0,18 mol) hinzugegeben wurden. Nach Lagerung bei 4 °C wurde der entstandene feste Niederschlag filtriert, mit Eiswasser (3 x 50 ml) und Ethanol (80 ml) gewaschen und im Vakuum zum Produkt getrocknet (18, 54,1 g, 75 % Ausbeute).

**BEISPIEL 18**

Alkylierung von 5-Acetylamino-2,4-6-triiod-3-{N-(2,3-dihydroxypropyl)}carbamoyl-Benzamid (18) zu: 5-{N-(2,3-Dihydroxypropyl)acetamido}-2,4,6-triiod-3-{N-(2,3-dihydroxypropyl)}carbamoyl-Benzamid (19)

Die Titelverbindung (18, 39,7 g, 0,059 mol) wurde in Propylenglycol (16,7 ml), Ethanol (120 ml), und 25 % Gew./Gew. Natriummethoxid (17,6 ml, 0,077 mol) gelöst. Man versetzte mit Chlorpropandiol (9,78 g, 0,0885 mol) und rührte die Mischung 1 h bei 25 °C. Das Reaktionsgemisch wurde auf 33 °C erwärmt und weitere 19 h gerührt; während dieser Zeit wurde 25 % Gew./Gew. Natriummethoxid (3,4 ml, 0,015 mol) zugegeben. Das Reaktionsgemisch wurde mit 12 M HCl abgeschreckt, im Vakuum destilliert, mit $H_2O$ (200 ml) rekonstituiert und nochmals zu einer wässrigen Lösung destilliert, die mit Dowex-Harz 50 $H^+$ Harz (62 g) und Duolite-Harz A-340 $OH^-$ (170 g) entionisiert wurde. Nach Eluierung der Harze mit $H_2O$ und Einengung erhielt man 150 g der Lösung, die mit Norit Ultra SX Holzkohle (1,0 g) 14 h bei 60 °C behandelt wurde. Nach Abfiltrieren der Holzkohle erhielt man eine wässrige Lösung, die 2 h mit Dowex 50 $H^+$ (1,0 g) und Duolite A-340 $OH^-$ (4 g) gerührt wurde. Das Harz wurde abfiltriert und die wässrige Lösung zu 50,3 g eines das Produkt (19, 32,8 g, 74 % Ausbeute) in Glyzerin/Propylenglycol enthaltenden Öls destilliert. Dieses Öl wurde nach dem im folgenden Schritt beschriebenen Verfahren gereinigt.

**BEISPIEL 19**

Peracetylierung, Reinigung über Siligagel und anschließende Deacetylierung von: 5-{N-(2,3-Dihydroxypropyl)acetamido}-2,4,6-triiod-3-{N-(2,3-dihydroxypropyl)}carbamoyl-Benzamid (19)

Die in Glyzerin/Propylenglycolöl (Gesamtmasse 25,15 g) gelöste Titelverbindung (19, 16,4 g, 0,022 mol) wurde mit Pyridin (1,74 g, 0,022 mol) und Essigsäureanhydrid (115 g, 1,12 mol) verdünnt und dann 18 h auf 60 °C erwärmt. Das Reaktionsgemisch wurde bei Unterdruck zu einem Öl destilliert, in $CHCl_3$ (100 ml) gelöst und mit 0,1 n HCl (2 x 50 ml) und 15 % Gew./Vol. Kochsalzlösung (2 x 50 ml) extrahiert. Die $CHCl_3$-Phase wurde über $MgSO_4$ getrocknet, filtriert, und zu einem Öl destilliert. Dieses Öl wurde an 900 g Silikagel unter Verwendung eines Lösungsmittelgradienten gereinigt, welcher von 5 % Essigsäure/95 % Chloroform bis 5 % Essigsäure/4 % Methanol/91 % Chloroform lief. Die gereinigten Fraktionen wurden zusammengefaßt, zu einem Schaum destilliert und dann mit Methanol (30 ml) und 25 % Gew./Gew. Natriummethoxid in Methanol (0,98 g, 0,0054 mol) behandelt. Nach 30 Minuten wurde die Lösung destilliert, mit Methanol (20 ml) rekonstituiert und dann mit Dowex-Harz 50 $H^+$ (1,3 g) gerührt. Nach Absenkung des pH-Werts von 12 auf 5 wurde das Harz abfiltriert, und man erhielt eine Lösung, die zu einem Schaum destilliert, mit $H_2O$ (25 ml) rekonstituiert und zu der festen Titelverbindung (19, 8,12 g, 49 % Ausbeute) eingedampft wurde.

**BEISPIEL 20**

Deacetylierung von 5-Diacetylamino-2,4,6-triiod-3-{N-(2,3-diacetoxypropyl)}carbamoyl-Benzoesäure (15) zu: 5-Acetylamino-2,4,6-triiod-3-{N-(2,3-dihydroxypropyl)}carbamoyl-Benzoesäure (20)

Die Titelverbindung (15, 720 g, 0,9 mol) wurde in 50 ml Methanol gelöst und mit 25 % Gew./Gew. Natriummethoxid in Methanol (345 ml, 1,5 mol) versetzt. Nach 4 h bei 45-50 °C wurde das Reaktionsgemisch bei Unterdruck destilliert und mit 12 M HCl (124 ml, 1,5 mol) angesäuert; die Salze wurden abfiltriert. Das Filtrat wurde bei Unterdruck destilliert, man erhielt ein Öl, das mit n-Propanol (680 ml) verdünnt wurde. Nach Kristallisierung bei 4 °C wurde das erhaltene feste Produkt (20) abfiltriert, mit n-Propanol (2 x 300 ml) gewaschen und im Vakuum getrocknet. Die Ausbeute betrug 391 g (64 %).

**BEISPIEL 21**

Alkylierung von 5-Acetylamino-2,4,6-triiod-3-{N-(2,3-dihydroxypropyl)}carbamoyl-Benzoesäure (20) zu: 5-{N-(2,3-Dihydroxypropyl)acetamido)-2,4,6-triiod-3-{N-)2,3-dihydroxypropyl)}carbamoylBenzoesäure-Natriumsalz (21)

Die Titelverbindung (20, 100 g, 0,148 mol) wurde in 400 ml Methanol gelöst. Man gab festes $Na_3PO_4$ . 12 $H_2O$ (140,6 g, 0,37 mol) und anschließend Chlorpropandiol (32,7 g, 0,296 mol) und 25 % Gew./Gew. Natriummethoxid in Methanol (24,1 g, 0,111 mol) tropfenweise hinzu. Das Reaktionsgemisch wurde 10 h auf 40 °C erwärmt, wobei weiteres 25 %-iges Natriummethoxid (8,0 g, 0,0368 mol) portionsweise zugegeben wurde. Die Salze wurden abfiltriert und das Methanolfiltrat wurde mit 12 M HCl (3,5 ml) angesäuert, im Vakuum zu einem dickflüssigen, öligen Produkt (21) eingeengt und direkt in die nächste Reaktionsstufe übernommen.

**BEISPIEL 22**

Acetylierung des Natriumsalzes der 5-{N-(2,3-dihydroxypropyl)acetamido}-2,4,6-triiod-3-{N-(2,3-dihydroxypropyl)}carbamoyl-Benzoesäure (21) zu: 5-{N-(2,3-Diacetoxypropyl)acetamido}-2,4,6-triiod-3-{N-(2,3-diacetoxypropyl)}carbamoyl-Benzoesäure (22)

Die Titelverbindung (21, 114 g, 0,148 mol) als Öl wurde mit Pyridin (11,7 g, 0,148 mol) und Essigsäureanhydrid (605 g, 5,92 mol) verdünnt und 2 h bei 65-70 °C gerührt. Das Reaktionsgemisch wurde zu einem Öl eingeengt, mit Butylacetat (2 x 100 ml) azeotrop destilliert und zwischen Wasser (300 ml) und 3 : 1 Toluol/Ethylacetat (200 ml) verteilt. Die wässrige Phase wurde mit 3 : 1 Toluol/Ethylacetat (3 x 100 ml) extrahiert und mit HCl (22,5 ml) in Gegenwart von Etylacetat (300 ml) angesäuert. Die angesäuerte $H_2O$-Phase wurde getrennt und zweimal mit Ethylacetat (100 ml) extrahiert. Die drei letzten Ethylacetatauszüge wurden vereinigt, über $MgSO_4$ getrocknet, filtriert und zu einem festen Produkt (22, 118 g, 87 % Ausbeute) eingedampft.

**BEISPIEL 23**

Chlorierung von 5-{N-(,3-diacetoxypropyl)-acetamido}-2,4,6-triiod-3-{N-(2,3-diacetoxypropyl)}carbamoyl-Benzoesäure (22) zu: 5-{N-(2,3-Diacetoxypropyl)acetamido}-2,4,6-triiod-3-{N-(2,3-diacetoxypropyl)}-carbamoyl-Benzoylchlorid (23)

Die Titelverbindung (22, 113,6 g, 0,124 mol) wurde bei 55 °C in Ethylacetat (100 ml) gelöst; man gab tropfenweise Thionylchlorid (44 g, 0,37 mol) hinzu und erhitzte das Gemisch 2 h unter Rückfluß, engte im Vakuum zu einem Öl ein und destillierte dann azeotrop mit Butylacetat (2 x 50 ml) zu einem Schaum, der in Chloroform (200 ml) gelöst und mit 0,2 M Phosphatpuffer pH 6,7 (100 ml) extrahiert wurde. Die organische Phase wurde über $MgSO_4$ getrocknet, filtriert und zu einem festen Produkt (23, 115 g, 98 % Ausbeute) eingedampft.

**BEISPIEL 24**

Amidierung von 5-{N-(2,3-Diacetoxypropyl)-acetamido}-2,4,6-triiod-3-{N-(2,3-diacetoxypropyl)}carbamoyl-Benzoylchlorid (23) zu: 5-{N-(2,3-Diacetoxypropyl)-acetamido}-2,4,6-triiod-3-{N-(2,3-diacetoxypropyl)-}carbamoyl-Benzamid (24)

Die Titelverbindung (23, 105 g, 0,111 mol) wurde in Acetonitril (400 ml) gelöst und unter Verwendung eines Trockeneiskühlers bei 25 °C mit wasserfreiem Ammoniak versetzt. Die Umsetzung war nach 3 h $NH_3$-Rückfluß abgeschlossen. Die Salze wurden abfiltriert, und nach Verdampfen erhielt man eine Festsubstanz (24, 98,8 g, 96 % Ausbeute).

**BEISPIEL 25**

Deacetylierung von 5-{N-(2,3-Diacetoxypropyl)-acetamido}-2,4,6-triiod-3-{N-(2,3-diacetoxypropyl)-}carbamoyl-Benzamid (24) zu: 5-{N-(2,3-Dihydroxypropyl)-acetamido}-2,4,6-triiod-3-{N-(2,3-dihydroxypropyl)}carbamoyl-Benzamid (19)

Die Titelverbindung (24, 98,7 g, 0,106 mol) wurde in Methanol (250 ml) gelöst, das bei 25 °C mit 25% Gew./Gew. Natriummethoxid in Methanol (2,30 g, 0,0106 mol) versetzt worden war. Nach 15 Minuten wurde die Lösung im Vakuum zu einem Öl eingeengt, mit Methanol (200 ml) rekonstituiert und dann mit Dowex-Harz 50 $H^+$ (6,0 g) gerührt bis der pH-Wert von 12 auf 6 gesunken war. Das Harz wurde abfiltriert, und man erhielt eine Lösung, die zu einem Schaum destilliert, mit Wasser (320 ml) und Norit SX Holzkohle (3,0 g) rekonstituiert, 7 h unter Rückfluß erhitzt, filtriert, durch Rühren mit Dowex-Harz 50 $H^+$ (3 g) und Dowex-Harz XUS-40123 $OH^-$ (12 g) entionisiert, filtriert und zu der Festsubstanz (19, 79,2 g, 96 % Ausbeute) eingedampft wurde.

**BEISPIEL 26**

Methoxyacetylierung von 5-Amino-2,4,6-triiod-3-{N-(1,3,4-trihydroxy-threo-but-2-yl)}carbamoyl-Benzamid (9) mit Methoxyacetylchlorid zu: 5-Methoxyacetylamino-2,4,6-triiod-3-{N-(1,3,4-trihydroxy-threo-but-2-yl)-}carbamoyl-Benzamid (25)

Die Titelverbindung (9, 100 g, 0,15 mol) wurde bei 25 °C in N,N-Dimethylacetamid (250 ml) suspendiert und über 30 Minuten mit Methoxyacetylchlorid (68 ml, 0,75 mol) versetzt. Das Reaktionsgemisch wurde 5 h bei 35 °C gerührt; die HPLC zeigte das Ende der Umsetzung an. Das Reaktionsgemisch wurde mit Natriummethoxid (97 g, 0,45 mol) abgeschreckt und 2 h bei 40 °C gerührt. Die Lösung wurde mit Dowex-Harz 50W-X4 neutralisiert, filtriert und mit n-Butanol (700 ml) verdünnt. Es bildete sich unmittelbar ein weißer Niederschlag, der zu einem weißlichen Feststoff (25, 80,6 g, 0,11 mol, 73 % Ausbeute) filtriert wurde.

**BEISPIEL 27**

Alkylierung von Ioxithalamsäure (26) zu: 5-{N-(2,3-Dihydroxypropyl)acetamido}-2,4,6-triiod-3-{N-(2-hydroxyethyl)}carbamoyl-Natriumbenzoat (27)

Ioxithalamsäure (26, 966 g, 1,5 mol) wurde bei Raumtemperatur in 1 n Natriumhydroxid (1,5 l) gelöst auf 75 °C erhitzt und innerhalb 1,25 h gleichzeitig mit 3-Chlor-1,2-propandiol (223,8 g, 2,03 mol) und 5 n Natriumhydroxid (ca. 0,4 l) versetzt. Das Reaktionsgemisch wurde für weitere 2,5 h auf 80-90 °C erhitzt, worauf HPLC den Abschluß der Umsetzung (ca. 90 %ige Umsetzung in das Produkt) anzeigte.
Das Reaktionsgemisch wurde mit konzentrierter Salzsäure (ca. 3 ml) neutralisiert und eingedampft. Etwa die Hälfte des schaumförmigen Rückstands wurde in Wasser (0,4 l) aufgenommen. Beim Abkühlen wurde eine weiße, kristalline Festsubstanz abgeschieden, die filtriert und mit Eiswasser gewaschen wurde. Nach Trocknung erhielt man das kristalline Produkt (27, 249 g).

**BEISPIEL 28**

Acetylierung von 5-{N-(2,3-Dihydroxypropyl)acetamido}-2,4,6-triiod-3-{N-(2-hydroxyethyl)}carbamoyl-Natriumbenzoat (27) zu: 5-{N-(2,3-Diacetoxypropyl)acetamido}-2,4,6-triiod-3-{N-(2-acetoxyethyl)}carbamoyl-Benzoesäure (28)

Die Titelverbindung (27, 50 g, 0,067 mol) wurde bei 25 °C in Essigsäureanhydrid (102 ml, 1,080 mol, 16,0 Äqu.) eingerührt. Pyridin (5,4 ml, 0,067 mol, 1,0 Äqu.) wurde zugegeben und die Temperatur 1 h auf 85 °C erhöht: die anschließende DSC zeigte den Abschluß der Umsetzung an. Das homogene Reaktionsgemisch wurde im Vakuum zu einem dickflüssigen Öl eingedampft, in Butylacetat (50 ml) gelöst und mehrfach eingedampft. Das Öl wurde in $H_2O$ (260 ml) gelöst und mit Toluol/Ethylacetat (2:1, 4 x 100 ml) extrahiert. Die wässrige Phase wurde mit 12 n Salzsäure (11 ml) angesäuert und mit Ethylacetat (3 x 50 ml) extrahiert. Die organische Phase wurde über Magnesiumsulfat getrocknet, zu einem Schaum (28) eingedampft und direkt in den nächsten Reaktionschritt übernommen (55 g, 0,065 mol, 97 % Ausbeute).

**BEISPIEL 29**

Chlorierung von 5-{N-(2,3-Diacetoxypropyl)acetamido}-2,4,6-triiod-3-{N-(2-acetoxyethyl)}carbamoyl-Benzoesäure (28) zu: 5-{N-(2,3-Diacetoxypropyl)acetamido}-2,4,6-triiod-3-{N-(2-acetoxyethyl)}carbamoyl-Benzoylchlorid (29)

Die Titelverbindung (28, 55 g, 0,065 mol) wurde in 1,2-Dichlorethan (170 ml) gelöst und auf 85 °C erhitzt. Man versetzte mit Thionylchlorid (9,8 ml, 0,134 mol, 2,0 Äqu.), und durch DSC wurde nachgewiesen, daß die Umsetzung nach 3 h abgeschlossen war. Das Reaktionsgemisch wurde im Vakuum zu einem Öl eingedampft, in Butylacetat (50 ml) nochmals gelöst und mehrfach eingedampft. Das Produkt wurde als gelber Schaum isoliert (29, 51,9 g, 0,060 mol, 92 % Ausbeute).

**BEISPIEL 30**

Amidierung von 5-{N-(2,3-Diacetoxypropyl)acetamido}-2,4,6-triiod-3-{N-(2-acetoxyethyl)}carbamoyl-Benzoylchlorid (29) zu: 5-{N-(2,3-Diacetoxypropyl)acetamido}-2,4,6-triiod-3-{N-(2-acetoxyethyl)}carbamoyl-Benzamid (30)

Die Titelverbindung (29, 51,9 g, 0,060 mol) wurde in Acetonitril (200 ml) gelöst und bei 10 °C mit wasserfreiem Ammoniak (Überschuß) versetzt. Nach 4 h zeigte die DSC den Abschluß der Umsetzung an. Das Reaktionsgemisch wurde filtriert, um die Ammoniumchloridsalze zu entfernen, und nach Entfernen des Lösungsmittels erhielt man ein gelbes Öl (30, 47 g, 0,056 mol, 93 % Ausbeute).

**BEISPIEL 31**

Deacetylierung von 5-{N-(2,3-Diacetoxypropyl)acetamido}-2,4,6-triiod-3-{N-(2-acetoxyethyl)}carbamoyl-Benzamid (30) zu: 5-{N-(2,3-Dihydroxypropyl)acetamido}-2,4,6-triiod-3-{N-(2-hydroxyethyl)}carbamoyl-Benzamid (31)

Die Titelverbindung (30, 47 g, 0,056 mol) wurde in Methanol (240 ml) gelöst und zur Erhöhung des pH-Werts auf ca. 12 mit 25 % Natriummethoxid (3,9 g, 0,3 Äqu.) versetzt. Die Lösung wurde 1 h bei 25 °C gerührt, worauf durch HPLC der Abschluß der Deacetylierung angezeigt wurde. Das Reaktionsgemisch wurde mit 1 n HCl (10 ml) neutralisiert, und nach Entfernung des Lösungsmittels erhielt man einen weißlichen Schaum (31, 39 g, 0,054 mol, 97 % Ausbeute, Reinheit 98 %), der aus erhitztem Methanol (5 g in 15 ml, mit Impfung) umkristallisiert wurde.

**BEISPIEL 32**

Amidierung von 5-Amino-2,4,6-triiod-isophthaloyl-Chlorid (32) zu: 5-Amino-2,4,6-triiod-3-chlorcarbonyl-Benzamid (33)

Die Ausgangssubstanz (32, 300 g, 0,503 mol) wurde in Tetrahydrofuran (900 ml) gelöst und die homogene Lösung in Eis auf 5-10 °C gekühlt. Innerhalb von 10 Minuten wurde konzentriertes Ammoniumhydroxid (92,3 ml, 1,38 mol) zugegeben; die Temperatur stieg auf 30 °C.
Das Reaktionsgemisch wurde bei Raumtempratur insgesamt 90 Stunden lang gerührt, wobei weiter Ammoniumhydroxid (insgesamt 25,2 ml, 0,38 mol) zugegeben wurde; dann wurde gekühlt, und die unlöslichen Salze wurden durch Filtrieren entfernt. Das Filtrat wurde mit gesättigter NaCl (200 ml x 2) gewaschen.
Nach Verdampfen des Tetrafuran erhielt man ein zähflüssiges Öl. Mit Ethylacetat (800 ml) wurde eine gelbbraune Festsubstanz ausgefällt, filtriert, mit Ethylacetat (100 ml x 2) gewaschen und getrocknet; man erhielt (33, 193 g, 66,5 % Ausbeute).

**BEISPIEL 33**

Dimerisierung von 5-Amino-2,4,6-triiod-3-chlorcarbonyl-Benzamid (33) zu: Bis-{(3-Chlorcarbonyl-5-carbamoyl)-2,4,6--triiodanilid}-Malonsäure (34)

Die Titelverbindung (33, 20,0 g, 34,7 mM) wurde in trockenem Tetrahydrofuran (100 ml) gelöst, auf 45 °C erhitzt und innerhalb von 3 Minuten mit Malonyldichlorid (2,53 ml, 26 mM) versetzt, wodurch man ein heterogenes Gemisch erhielt. Man setzte trockenes THF (100 ml) zu und ließ die Suspension 1 h rühren, worauf die DSC anzeigte, daß die Umsetzung abgeschlossen war. Das Gemisch wurde mit Butylacetat (150 ml) verdünnt, die Festsubstand wurde filtriert, mit Butylacetat (50 ml x 2) gewaschen und im Vakuum zum Produkt (34, 13,18 g, 62 % Ausbeute) getrocknet.

**BEISPIEL 34**

Amidierung von Bis-{(3-chlorcarbonyl-5-carbamoyl)-2,4,6--triiodanilid}-Malonsäure (34) zu: bis-[{3-N-(1,3,4-Trihydroxy-threo-but-2-yl-carbamoyl-5-carbamoyl}-2,4,6-triiodanilid]-Malonsäure (35)

Die Titelverbindung (34, 8,0 g, 6,56 mM) wurde in trockenem N,N-Dimethylacetamid (10 ml) gelöst, mit Triethylamin (1,83 ml, 13,12 mM) versetzt und die Lösung auf 20 °C gekühlt. Über 3 Minuten wurde trans-5-Amino-2,2-dimethyl-6-hydroxy-1,3-dioxepan (2,64 g, 16,4 mM) zugesetzt und das homogene Gemisch 6 h bei Raumtemperatur gerührt, worauf die DSC den Abschluß der Umsetzung anzeigte. Das Lösungsmittel wurde verdampft, Wasser (50 ml) wurde zugesetzt und das Gemisch 15 Minuten auf 75 °C erhitzt, um die Acetonide abzuspalten.. Nach Eindampfen und Ausfällen mit Isopropanol (100 ml) erhielt man das Produkt. Der Feststoff wurde filtriert, mit Isopropanol (20 ml x 2) gewaschen und getrocknet. Man erhielt 8,6 g (35, 94 % Ausbeute).

**BEISPIEL 35**

Amidierung von 5-N-Methylamino-2,4,6-triiod-isophthaloyl Chlorid (36) zu: 5-N-Methylamino-2,4,6-triiod-3-chlorcarbonyl-Benzamid (37)

Die Ausgangssubstanz (36, 305 g, 0,5 mol) wurde in Tetrahydrofuran (1 l) gelöst und auf 10 °C gekühlt. Über 5 Minuten wurde konzentriertes Ammoniumhydroxid (100 ml, 1,5 mol) hinzugegeben; die Temperatur stieg auf ca. 25 °C.

Man ließ das Reaktionsgemisch bei Raumtemperatur 65 Stunden rühren, wobei nach 20 Stunden (3,5 ml) und 44 Stunden (3,5 ml) weitere konzentrierte $NH_4OH$ zugegeben wurde.

Nach dem Abkühlen wurden die unlöslichen Salze und bis-Amide abfiltriert und das THF-Filtrat mit gesättigter Natriumchloridlösung (100 ml x 2) gewaschen. Das THF wurde verdampft und das Produkt mit Ethylacetat (500 ml) aus dem dickflüssigen Öl ausgefällt. Nach Filtrierung, Waschen mit Ethylacetat und Trocknen erhielt man (37, 132,1 g, 45 % Ausbeute).

**BEISPIEL 36**

Dimerisierung von 5-N-Methylamino-2,4,6-triiod-3-chlorcarbonyl-Benzamid (37) zu: Bis-{(3-Chlorcarbonyl-5-carbamoyl)-2,4,6-triiod-N-methyl Anilid}-Malonsäure (38)

Die Titelverbindung (37, 25 g, 42,3 mM) wurde in trockenem Tetrahydrofuran (100 ml) gelöst und die homogene Lösung auf 50 °C erhitzt. Über 2 Minuten wurde Malonyldichlorid (3,05 ml, 31,3 mM) und anschließend weiteres Tetrahydrofuran (50 ml) zugesetzt und die Suspension 1 h erhitzt, woraufhin die DSC

den Abschluß der Umsetzung anzeigte.

Nach Verdünnen mit Butylacetat (50 ml) wurde das Produkt filtriert, mit Butylacetat (25 ml x 2) gewaschen und getrocknet; man erhielt eine weißliche Festsubstanz (38, 15,24 g, 58 % Ausbeute).

**BEISPIEL 37**

Umwandlung von Bis-{(3-Chlorcarbonyl-5-carbamoyl)-2,4,6-triiod-N-methyl Anilid}-Malonsäure (38) in: Bis-[{3-N-(1,3,4-Trihydroxy-threo-but-2-yl-carbamoyl)-5-carbamoyl}-2,4,6-triiod-N-methyl-Anilid]-Malonsäure (39)

Die Ausgangssubstanz (38, 10 g, 8 mM) wurde in trockenem N,N-Dimethylacetamid (15 ml) und Triethylamin (2,23 ml, 16 mM) gelöst. Über 5 Minuten wurde trans-5-Amino-2,2-dimethyl-6-hydroxy-1,3-dioxepan (Aminodioxepan) (3,22 g, 20 mM) zugesetzt, und das homogene Gemisch wurde 8 h gerührt, worauf durch DSC nachgewiesen wurde, daß die Umsetzung abgeschlossen war.
Die DMA wurde durch Destillation im Vakuum entfernt, und die Isopropylidene wurden bei 50 °C mit wässriger Salzsäure gespalten. Das Wasser wurde im Rotationsverdampfer entfernt, und zur Ausfällung des Produkts wurde Isopropanol zugesetzt. Nach Filtrieren, Waschen mit Isopropanol (10 ml x 3) und Trocknen erhielt man das Dimer (39, 9,86 g, 87 % Ausbeute).

**BEISPIEL 38**

Umwandlung von 5-N-(Methyl)amino-2,4,6-triiod-3-chlorcarbonyl-Benzamid (36) in: 5-{N-(Methyl)-2-acetoxyacetamido}-2,4,6-triiod-3-chlorcarbonyl-Benzamid (40)

Die Ausgangssubstanz (36, 25 g, 42,3 mM) wurde bei Raumtemperatur in N,N-Dimethylacetamid (50 ml) gelöst. Man setzte 2-Acetoxyacetyl-Chlorid (6,83 ml, 63,5 mM) zu und ließ über Nacht rühren; die anschließende DSC zeigte den Abschluß der Umsetzung an.
Durch Zugabe von Eiswasser (200 ml) wurde das Produkt ausgefällt und filtriert. Nach Waschen mit Wasser wurde der Feststoff in Tetrahydrofuran (200 ml) gelöst und die Lösung mit gesättigter NaCl:gesättigter $NaHCO_3$ (3:1, 250 ml) und nochmals mit gesättigter NaCl (100 ml) extrahiert. Die organische Phase wurde getrocknet ($MgSO_4$) und das Lösungsmittel entfernt; man erhielt einen Schaum (40, 22,1 g, 77,2 % Ausbeute).

**BEISPIEL 39**

Amidierung und Spaltung von 5-{N-(Methyl)-2-acetoxyacetamido}-2,4,6-triiod-3-chlorcarbonyl Benzamid (40) zu: 5-{N-(Methyl)-2-hydroxyacetamido}-2,4,6-triiod-3-{N-(1,3,4-trihydroxy-threo-but-2-yl)}-carbamoyl-Benzamid (41)

Die Titelverbindung (40, 7,0 g, 10,35 mM) wurde in einem Gemisch von Tetrahydrofuran (40 ml) und Triethylamin (1,44 ml, 10,35 mM) gelöst und auf 10 °C gekühlt. Man versetzte mit festem Aminodioxepan (2,0 g, 12,41 mM), die Kühlung wurde beendet, und man ließ das Reaktionsgemisch bei 25 °C rühren. Nach 18 h zeigte die DSC den Abschluß der Umsetzung an. Das Reaktionsgemisch wurde mit Tetrahydrofuran (40 ml) und gesättigter NaCl:gesättigter $NaHCO_3$ (3:1, 50 ml) verdünnt und die Phasen getrennt. Die organische Phase wurde mit gesättigter NaCl (40 ml x 2) gewaschen, getrocknet und das Lösungsmittel verdampft; man erhielt einen Schaum (6,9 g, 82 % Ausbeute).
Der Schaum wurde in Methanol (50 ml) und mit 4,6 normaler NaOMe-Lösung (0,5 ml) versetzt. Die Lösung wurde bei 50 °C gestrippt; man erhielt ein Öl, das anschließend mit Wasser (50 ml) und Dowex-Harz 50 $H^+$ (10 g) gemischt wurde. Nach Erhitzen für 30 Minuten auf 60 °C erhielt man schließlich eine homogene Lösung, und die HPLC zeigte, daß die Ester- und Isopropyliden-Spaltung abgeschlossen war.

Das Harz wurde abfiltriert und die Lösung nacheinander über Duolite A 340 $OH^-$/Dowex 50 $H^+$-Säulen gegeben, bis die Deionisierung abgeschlossen war. Die Verbindung wurde mit Wasser aus den Säulen eluiert und anschließend mit Norit Ultra S-X Kohle (0,4 m) behandelt. Nach 1 h bei 70 °C wurde die Kohle abfiltriert und das Wasser verdampft; man erhielt einen weißen Schaum (41, 3,8 g, 50 % Ausbeute von 40).

**Beispiel 40**

Acetoxyacetylierung von 5-Amino-2,4,6-triiod-3-chlorcarbonyl-benzamid (33) zu:
5-Acetoxyacetylamino-2,4,6-triiod-3-chlorcarbonyl-benzamid (42)

Das Ausgangsmaterial (33, 200 g, 0,347 mol) wurde in 1200 ml Dioxan auf 60° C erhitzt. Acetoxyacetylchlorid (142 g, 1,041 mol) wurde innerhalb von 15 Minuten tropfenweise zugefügt, währenddessen die Reaktionstemperatur auf 90° C erhöht wurde. Man behielt diese Temperatur für 6,5 Stunden bei. Nach Abkühlen auf 15° C wurde das Festprodukt (42) abfiltriert, mit 4 mal 100 ml Dioxan gewaschen und im Vakuum getrocknet. (Ausbeute 200,5 g, 85 %)

**Beispiel 41**

Amidierung von 5-Acetoxyacetylamino-2,4,6-triiod-3-chlorcarbonyl-benzamid (42) zu 5-Acetoxyacetylamino-2,4,6-triiod-3-[-N-(2,3-dihydroxypropyl)]-carbamoyl-benzamid (43)

Das Ausgangsmaterial (42, 118 g, 0,174 mol) wurde zu N,N-Dimethylacetamid (180 ml), 3-Amino-1,2-propandiol (24,2 g, 0,266 mol) und Triethylamin (18,0 g, 0,177 mol) gegeben. Die Reaktion wurde 24 Stunden lang auf 25° C gehalten und dann durch tropfenweises Zufügen von n-Pentanol (1080 ml) unter heftigem Rühren verdünnt. Der erhaltene Niederschlag (43) wurde abfiltriert, mit 4 mal 100 ml n-Pentanol gewaschen und im Vakuum getrocknet. Man erhielt 124,9 g (Rohausbeute 98%).

**Beispiel 42**

Deacetylierung von 5-Acetoxyacetylamino-2,4,6-triiod-3-[-N-(2,3-dihydroxypropyl)]-carbamoyl-benzamid (43) zu 5-Hydroxyacetylamino-2,4,6-triiod-3-[N-(2,3-dihydroxypropyl)]-carbamoyl-benzamid (44)

Das Ausgangsmaterial (43, 124,8 g, 0,170 mol) wurde in Methanol (1,5 l) und Wasser (0,5 l) gelöst und dann mit Dowex 50 $H^+$ und Biorex 5 $OH^-$ Ionenaustauscherharz behandelt. Die Harze wurden nach 20-stündigem Rühren mit einem Sieb entfernt und das so erhaltene Gemisch wurde unter verminderten Druck zu einem Feststoff destilliert. Methanol (400 ml) und 25 %iges w/w Natriummethoxid in Methanol (36,9 g, 0,17 mol) wurden zu dem Rückstand gegeben. Die so erhaltene Lösung wurde anschließend filtriert und das Methylacetat abdestilliert. Man verdünnte mit Methanol, neutralisierte mit konz. HCL und destillierte unter verminderten Druck um einen Feststoff zu erhalten, der aus 9,9 g NaCl und 94,9 g des Produktes (44) bestand. Die Ausbeute betrug 80 %.

**Beispiel 43**

Umwandlung von 5-Hydroxyacetylamino-2,4,6-triiod-3-[N-(2,3-dihydroxypropyl)]carbamoyl-benzamid (44) mit 3,4-Dihydro-2H-pyran in 5-(2-Tetrahydropyranyloxy)acetylamino-2,4,6-triiod-3-[N-(2,3-tetrahydropyranyloxy)propyl]carbamoyl-benzamid (45)

Das Ausgangsmaterial (44, 3,44 g, 5 mMol) wurde mit Dioxan (15 ml) und Methansulfonsäure (29,6 mg, 0,31 mMol) gemischt. 3,4-Dihydro-2H-pyran (3,36 g, 40 mMol) wurden zugefügt und die Mischung wurde 4 Tage lang bei 25° C gerührt. Die Reaktionslösung wurde filtriert, mit Triethylamin (62 mg, 0,62 mMol) versetzt unter verminderten Druck zu einem Öl destilliert, mit Methanol aufgenommen und unter verminderten Druck zu einem Feststoff (45) destilliert, der direkt in die nachfolgende Alkylierung eingesetzt wurde.

**Beispiel 44**

Alkylierung und Spaltung von 5-(2-Tetrahydropyranyloxy)acetylamino-2,4,6-triiod-3-[N-(2,3-tetrahydropyranyloxy)propyl]carbamoyl-benzamid (45) zu 5-[N-(2-Hydroxyethyl)hydroxyacetamido]-2,4,6-triiod-3-[N-(2,3-dihydroxypropyl)]carbamoyl-benzamid (46)

Das Ausgangsmaterial (45, 5,0 mMol), ein halbfester Rückstand aus der vorhergehenden Reaktionsstufe, wurde mit Methanol (18 ml), Trinatriumphosphat-dodecahydrat (4,75 g, 12,5 mMol) und Chlorethanol (805 mg, 10 mMol) gemischt. Die so erhaltene Suspension wurde 31 Stunden lang bei 40 bis 45° C gerührt, dann filtriert und mit 0,5 ml konz. HCl versetzt. Das angesäuerte Filtrat wurde unter reduzierten

Druck zu einem Öl eingeengt und mit 0,01 n HCl (20 ml) und Methanol (20 ml) wieder aufgenommen. Nach zweimaliger Wiederholung dieses Verfahrens wurde die saure Lösung unter reduziertem Druck zu einem Festprodukt (46, 3,50 g, Ausbeute 95 %) eingeengt.

**Beispiel 45**

Alkylierung von 3,5-Diacetylamino-2,4,6-triiod-benzoesäure (Diatrizoicsäure, 47) zu Natrium-3,5-[N,N'-(2,3-dihydroxypropvl]diacetamido]-2,4,6-triiod-benzoat (48)

Eine Suspenion des Ausgangsmaterials (47, 50 g 0,079 mol) in Methanol (300 ml) wurde mit Trinatriumphosphat-dodecahydrat (149 g, 0,393 mol) und 3-Chlor-1,2-propandiol (35 g, 0,314 mol) versetzt und die Reaktion 24 Stunden auf 40° C erhitzt. Unlösliche Salze wurden durch Vakuumfiltration entfernt, das Filtrat wurde mit HCl neutralisiert und zu einem weißen Schaum (48, ca. 59 g, Ausbeute 94 % einschließlich 10 % Ester-Nebenprodukt) eingeengt. Das Produkt wurde direkt in die nachfolgende Acetylierung eingesetzt.

**Beispiel 46**

Acetylierung von Natrium-3,5-[N,N'-(2,3-dihydroxypropyl)diacetamido]-2,4,6-triiod-benzoat (48) zu 3,5-[N,N'-(2,3-Diacetoxypropyl)diacetamido]-2,4,6-triiod-benzoesäure (49)

Das Ausgangsmaterial (48, 59 g, 0,075 mol) wurde mit Acetanhydrid (150 ml, 1,58 mol) und Pyridin (6 ml, 0,075 mol) gemischt und 1 Stunde auf 85° C erhitzt. Acetanhydrid, Essigsäure und Pyridin wurden durch Destillation bei 70 bis 80° C entfernt. Der gelbe Schaum wurde mit Butylacetat (50 ml x 2) azeotrop destilliert. Das Produkt (Natriumsalz von 49) wurde in Wasser (300 ml) gelöst und mit einem 2:1 Gemisch von Toluol und Ethylacetat (150 ml x 3) extrahiert, um das Esternebenprodukt der vorhergehenden Alkylierungsstufe zu entfernen. Die wässrige Lösung wurde mit konz. HCl zu pH 2,5 angesäuert. Der weiße Niederschlag wurde mit Ethylacetat (75 ml x 2) extrahiert. Die vereinigten organischen Extrakte wurden über Magnesiumsulfat getrocknet. Das Lösungsmittel wurde entfernt. Man erhielt ein gelbes Öl (49, ca. 58 g, 83 % Gesamtausbeute aus 47), das direkt in die nachfolgende Chlorierung eingesetzt wurde.

**Beispiel 47**

Chlorierung von 3,5-[N,N'-(2,3-Diacetoxypropyl)diacetamid]-2,4,6-triiod-benzoesäure (49) zu 3,5-[N,N'-(2,3-diacetoxypropyl)diacetamido]-2,4,6-triiod-benzoylchlorid (50)

Das Ausgangsmaterial (49, ca. 0,062 mol), gelöst in Ethylacetat (125 ml), wurde mit Thionylchlorid (23 ml, 0,32 mol) bei 65 bis 70° C versetzt. Man erhöhte die Temperatur eine Stunde lang auf 75 bis 80° C. Die DSC zeigte an, daß die Reaktion vollständig war, sodaß man Thionylchlorid und Ethylacetat im Vakuum entfernte. Der Rückstand wurde mit Butylacetat (150 ml x 2) azeotrop destilliert und der erhaltene Feststoff getrocknet. Der gelbbraune Schaum (50, ca. 56 g, Ausbeute 95%) wurde direkt in die nachfolgende Amidierungsstufe eingesetzt.

**Beispiel 48**

Amidierung von 3,5-[N,N'-(2,3-diacetoxypropyl)diacetamido]-2,4,6-triiod-benzoylchlorid (50) zu 3,5-[N,N'-(2,3-Diacetoxypropyl)diacetamido]-2,4,6-triiod-benzamid (51)

Das in Acetonitril (200 ml) gelöste Ausgangsmaterial (50, ca. 0,059 mol) wurde mit wasserfreiem Ammoniak (Überschuß) bei 10° C versetzt. Die Temperatur wurde 5 Stunden lang auf 25° C erhöht. Danach war die Reaktion laut DSC vollständig. Das Ammoniumchlorid wurde durch Filtration entfernt und das Filtrat zu einem gelben Schaum (51, 53 g, geschätzte Ausbeute 97 %) eingeengt. Der Schaum wurde direkt in die nachfolgende Acetylierung eingesetzt.

**Beispiel 49**

Deacetylierung von 3,5-[N,N'-(2,3-Diacetoxypropyl)diacetamido]-2,4,6-triiod-benzamid (51) zu 3,5-[N,N'-(2,3-Dihydroxypropyl)diacetamido]2,4,6-triiod-benzamid (52)

Eine Lösung des Ausgangsmaterials (51, ca. 0,057 mol) in Methanol (250 ml) wurde mit Natriummethoxid (4,6 molar, 5,0 g, 0,023 mol) gemischt und bei Raumtemperatur 30 Minuten lang gerührt. HPLC zeigte danach, daß die Deacetylierung vollständig war. Das Reaktionsgemisch wurde mit konz. HCl neutralisiert. Das unlösliche Natriumchlorid wurde durch Filtration entfernt und das Filtrat zu einem gelben Schaum (52, 43 g, Ausbeute 97 %, Reinheit 98 % laut HPLC) eingeengt.

**BEISPIEL** 50

Injektionslösungen enthaltend 5-{N-(2,3-dihydroxypropyl)acetamido}-2,4,6-triiod-3-{N-(2,3-dihydroxypropyl)-}carbamoyl-Benzamid (19)

| Zusammensetzung von 100 ml Aliquote der Lösung | Jodgehalt der Injektionslösung in mg/ml | | |
|---|---|---|---|
| | 300 | 350 | 400 |
| Verbindung (g) | 58,87 | 68,68 | 78,49 |
| Dinatrium, Kalziumsalz der Ethylendiamintetraessigsäure (mg) | 10 | 10 | 10 |
| tris-(Hydroxymethyl)aminomethan (mg) | 121 | 121 | 121 |
| Wasser, injektionsrein, zum Auffüllen (ml) | 100 | 100 | 100 |
| Osmolalität (mOsm/kg) | 399 | 473 | 510 |
| Viskosität bei 37 °C (Centipoise) | 4,1 | 6,6 | 10,6 |

Verfahrensweise: Das Natrium-Kalzium-Salz der Ethylendiamintetraessigsäure, tris-(Hydroxymethyl)-aminomethan und das Kontrastmittel wurden in injektionsreinem Wasser gelöst und durch Versetzen mit 1 n Salzsäure auf pH 7,0 eingestellt. Die Lösungen wurden mit injektionsreinem Wasser auf 100 ml aufgefüllt, über eine 0,22 µm Membran in Glasampullen filtriert, verschlossen und 20 Minuten bei 121 °C im Autoklaven behandelt.

**BEISPIEL** 51

Injektionslösungen enthaltend 5-{N-(2-hydroxyethyl)acetamido}-2,4,6-triiod-3-{N-(1,3,4-trihydroxy-threo-but-2-yl)}carbamoyl-Benzamid (11)

| Zusammensetzung von 100 ml Aliquote der Lösung | Jodgehalt der Injektionslösung in mg/ml | | |
|---|---|---|---|
| | 300 | 350 | 400 |
| Verbindung (g) | 58,87 | 68,68 | 78,49 |
| Dinatrium, Kalziumsalz der Ethylendiamintetraessigsäure (mg) | 56 | 56 | 56 |
| Trinatriumcitrat (mg) | 77 | 77 | 77 |
| Wasser, injektionsrein, zum Auffüllen (ml) | 100 | 100 | 100 |
| Osmolalität (mOsm/kg) | 301 | 337 | 370 |
| Viskosität bei 37 °C (Centipoise) | 4,2 | 6,6 | 13,1 |

Verfahrensweise: Das Kalzium-Dinatrium-Salz der Ethylendiamintetraessigsäure, Trinatriumcitrat und das Kontrastmittel wurden in injektionsreinem Wasser gelöst und durch Versetzen mit Natriumkarbonat und Kohlensäure auf pH 5,0 bis 6,0 eingestellt. Die Lösungen wurden mit injektionsreinem Wasser auf 100 ml aufgefüllt, über eine 0,22 µm Membran in Glasampullen filtriert, verschlossen und 20 Minuten bei 121 °C im Autoklaven behandelt.

Die vorstehenden Ergebnisse zeigen deutlich, daß neuartige nicht-ionische Kontrastmittel zur Verfügung gestellt werden, die gegenüber den zur Zeit erhältlichen Verbindungen wesentlich verbesserte Eigenschaften aufweisen. Aufgrund der Verbesserung der physikalischen Eigenschaften, insbesondere der Osmolalität und der Viskosität, kann bei leichter Verabreichung und größerer Schmerzfreiheit ein weiter Bereich von Körperregionen untersucht werden. Trotz der großen Anzahl von Verbindungen, die bereits hergestellt und erprobt wurden, hat sich herausgestellt, daß die erfindungsgemäßen Verbindungen den bisher offenbarten Verbindungen überlegen sind. Die neuartigen Eigenschaften werden durch Bereitstellung von drei verschiedenen Stickstoffgruppen im Molekül, von denen nur zwei substituiert sind, erreicht. Außerdem werden wirksame und hohe Ausbeuten liefernde Synthesewege angegeben, wobei leicht verfügbare Substanzen eingesetzt werden können.

Obwohl die vorstehende Erfindung zum besseren Verständnis durch Erläuterungen und Beispiele ziemlich detailliert beschrieben wurde, wird jedem mit normalen Fachkenntnissen Ausgestatteten anhand der in dieser Erfindung enthaltenen Lehren klar sein, daß gewisse Änderungen und Abwandlungen möglich sind, ohne den Inhalt oder Umfang der beigefügten Patentansprüche zu verlassen.

**Patentansprüche**

**1.** Nicht-ionisches Kontrastmittel der Formel I

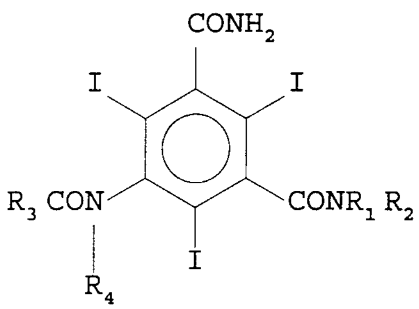

(I)

mit mindestens zwei Hydroxygruppen;
worin

$R_1$ Wasserstoff, ein niederes Alkyl oder Hydroxy-substituiertes niederes Alkyl bedeutet, wobei die Alkylgruppe zwischen 1-6 Kohlenstoffatome aufweist;

$R_2$ ein Hydroxyalkyl mit 2-6 C-Atomen bedeutet, das 1 bis n-1 Hydroxylgruppen aufweist, wobei n die Anzahl der Kohlenstoffatome ist;

$R_3$ ein niederes Alkyl, Hydroxy-substituiertes niederes Alkyl oder Alkoxy-substituiertes niederes Alkyl mit 1-6 C-Atomen, oder ein Rest der Formel II

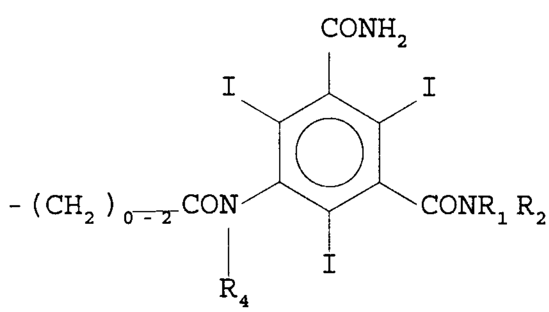

(II)

bedeutet und

$R_4$ Wasserstoff oder ein Alkyl mit 1-6 C-Atomen bedeutet, das 0 bis n-1 Hydroxylgruppen aufweist, wobei n die Anzahl der Kohlenstoffatome ist,

mit Ausnahme des 3-[N-(1,3-Dihydroxyprop-2-yl)carbamoyl] 5-(2-Hydroxy-1-oxopropyl) amino 2,4,6,-triiod-benzamids

**2.** Nicht-ionisches Kontrastmittel gemäß Anspruch 1, dadurch gekennzeichnet, daß $R_2$ 4-C-Atome und drei Hydroxylgruppen aufweist, $R_3$ nicht für einen Rest der Formel II steht und $R_4$ 2-3 C-Atome und mindestens eine Hydroxylgruppe aufweist.

**3.** Nicht-ionisches Kontrastmittel gemäß Anspruch 1, dadurch gekennzeichnet, daß $R_2$ 3 C-Atome und 2 Hydroxylgruppen aufweist, $R_3$ nicht für einen Rest der Formel II steht und $R_4$ 3 C-Atome und 2 Hydroxylgruppen aufweist.

**4.** Nicht-ionisches Kontrastmittel gemäß Anspruch 3, dadurch gekennzeichnet, daß $R_2$ und $R_4$ gleich sind.

**5.** 5-{N-(2-hydroxyethyl)acetamido)}-2,4,6-triiod-3-{N-(1,3,4-trihydroxy-threo-but-2yl)}carbamoyl-Benzamid.

5-{N-(2,3-Dihydroxypropyl)acetamido}-2,4,6-triiod-3-{N-(2,3-dihydroxypropyl)}carbamoyl-Benzamid,

5-{N-(2,3-Dihydroxypropyl)acetamido}-2,4,6-triiod-3-{N-(2-hydroxyethyl)}carbamoyl-Benzamid,

5-{N-(Methyl)-2-hydroxyacetamido}-2,4,6-triiod-3-{N-(1,3,4-trihydroxy-threo-but-2-yl)}carbamoyl-Benzamid,

Bis-[3-{N-(1,3,4-Trihydroxy)-threo-but-2-yl}carbamoyl-5-carbamoyl]-2,4,6-triiod-N-(methyl)anlilid-Malonsäure,

Bis-[{3-N-(2,3-Dihydroxypropyl-carbamoyl)5-carbamoyl}-2,4,6-triiodo-N-(2,3-dihydroxypropyl)anilid-Malonsäure;

Bis-[{3-N-(2,3-Dihydroxypropyl-carbamoyl)5-carbamoyl}-2,4,6-triiodo-N-(2-hydroxyethyl)anilid-Malonsäure;

5-[N-(2-Hydroxyethyl)hydroxyacetamido]-2,4,6-triiod-3-[N-(2,3-dihydroxypropyl)]carbamoyl-benzamid,

3,5-[N,N'-(2,3-Dihydroxypropyl)diacetamido]-2,4,6-triiod-benzamid.

**6.** Ein röntgenologisches Mittel bestehend aus einem nicht-ionischen Kontrastmittel gemäß Anspruch 1 in einem physiologisch verträglichen Träger.

**7.** Ein röntgenologisches Mittel bestehend aus einem nicht-ionischen Kontrastmittel gemäß Anspruch 5 in einem physiologisch verträglichen Träger.

**8.** Ein röntgenologisches Mittel bestehend aus einem nicht-ionischen Kontrastmittel gemäß Anspruch 6 in einem physiologisch verträglichen Träger.

**9.** Verfahren zur nicht-invasiven Bestimmung eines physiologischen Zustands unter Verwendung von Strahlen und einem nicht-ionischen Kontrastmittel, dadurch gekennzeichnet, daß man ein nicht-ionischen Kontrastmittel nach Anspruch 1 verwendet.

**10.** Verfahren zur Herstellung von Verbindungen der allgemeinen Formel I dadurch gekennzeichnet, daß man

a) Verbindungen der allgemeinen Formel V

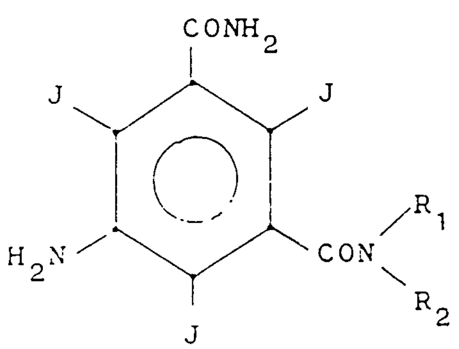

(V),

worin in $R_1$ und $R_2$ enthaltene Hydroxygruppen gegebenenfalls geschützt sind, durch Umsetzung mit $R_3$CO-X, wobei X für Halogen oder einen Esterrest steht, in katalysierenden

Losungsmitteln wie z.B. Pyridin, DMA oder DMF und gegebenenfalls anschließender Abspaltung der Schutzgruppen in Endprodukte der allgemeinen Formel I mit $R_4$ in der Bedeutung von Wasserstoff oder in Zwischenprodukte der allgemeinen Formel I mit $R_4$ in der Bedeutung von $R_3CO$ überführt und diese, gegebenenfalls mit geschützten Hydroxygruppen, anschließend gewünschtenfalls durch Alkylierung unter basischen Bedingungen mit $R_4$-enthaltenden Reagenzien, gegebenenfalls nach folgender Abspaltung der Schutzgruppen, in Endprodukte der allgemeinen Formel I, in welcher $R_4$ nicht Wasserstoff bedeutet, umwandelt bzw.

b) Verbindungen der allgemeinen Formel VI

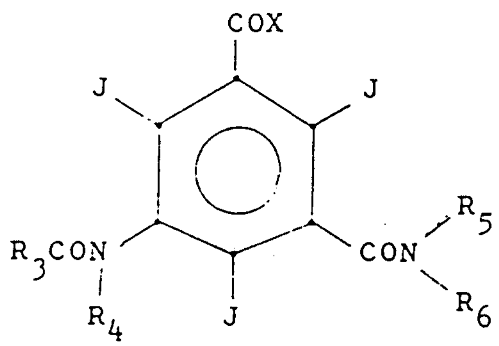

(VI)

mit $R_5$ in der Bedeutung von $R_1$ oder Wasserstoff, $R_6$ in der Bedeutung von $R_2$ oder Wasserstoff und X in der Bedeutung von Halogen oder eines Esterrestes,

mit Ammoniak bzw. mit Reste $R_1$ und $R_2$ enthaltenen Hydroxyalkylaminen, deren Hydroxygruppen geschützt sein können, umsetzt und anschließend gegebenenfalls die Schutzgruppen abspaltet.

**Claims**

**1.** Non-ionic contrast medium of formula I

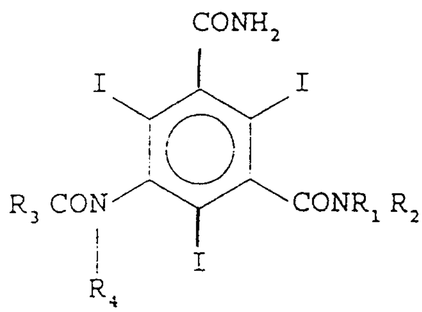

(I)

having at least two hydroxy groups;

wherein

$R_1$ represents hydrogen, lower alkyl or hydroxy-substituted lower alkyl, the alkyl group containing from 1 to 6 carbon atoms,;

$R_2$ represents hydroxyalkyl containing from 2 to 6 carbon atoms that has from 1 to n-1 hydroxy groups, n representing the number of carbon atoms;

$R_3$ represents lower alkyl, hydroxy-substituted lower alkyl or alkoxy-substituted lower alkyl containing from 1 to 6 carbon atoms, or a radical of formula II

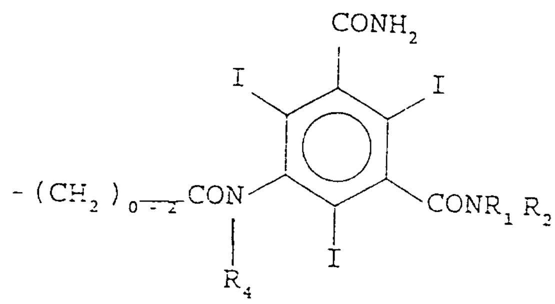

(II)

and

$R_4$ represents hydrogen or alkyl containing from 1 to 6 carbon atoms that has from 0 to n-1 hydroxy groups, n representing the number of carbon atoms,

with the exception of 3-[N-(1,3-dihydroxyprop-2-yl)carbamoyl]-5-(2-hydroxy-1-oxopropyl)amino-2,4,6-triiodo-benzamide.

2. Non-ionic contrast medium according to claim 1, characterised in that $R_2$ contains 4 carbon atoms and three hydroxy groups, $R_3$ does not represent a radical of formula II and $R_4$ contains 2 or 3 carbon atoms and at least one hydroxy group.

3. Non-ionic contrast medium according to claim 1, characterised in that $R_2$ contains 3 carbon atoms and 2 hydroxy groups, $R_3$ does not represent a radical of formula II and $R_4$ contains 3 carbon atoms and 2 hydroxy groups.

4. Non-ionic contrast medium according to claim 3, characterised in that $R_2$ and $R_4$ are the same.

5. 5-{N-(2-hydroxyethyl)acetamido}-2,4,6-triiodo-3-{N-(1,3,4-trihydroxythreobut-2-yl)}carbamoyl benzamide,
5-{N-(2,3-dihydroxypropyl)acetamido}-2,4,6-triiodo-3-{N-(2,3-dihydroxypropyl)}carbamoyl benzamide,
5-{N-(2,3-dihydroxypropyl)acetamido}-2,4,6-triiodo-3-{N-(2-hydroxyethyl)}carbamoyl benzamide,
5-{N-(methyl)-2-hydroxyacetamido}-2,4,6-triiodo-3-{N-(1,3,4-trihydroxythreobut-2-yl)}carbamoyl benzamide,
malonic acid-bis-[3-{N-(1,3,4-trihydroxy)threobut-2-yl}carbamoyl-5-carbamoyl]-2,4,6-triiodo-N-(methyl)anilide,
malonic acid-bis-[{3-N-(2,3-dihydroxypropylcarbamoyl)-5-carbamoyl}-2,4,6-triiodo-N-(2,3-dihydroxy propyl)-anilide,
malonic acid-bis-[{3-N-(2,3-dihydroxypropylcarbamoyl)-5-carbamoyl}-2,4,6-triiodo-N-(2-hydroxyethyl)anilide,
5-[N-(2-hydroxyethyl)hydroxyacetamido]-2,4,6-triiodo-3-[N-(2,3-dihydroxypropyl)]carbamoyl benzamide,
3,5-[N,N'-(2,3-dihydroxypropyl)diacetamido)]-2,4,6-triiodobenzamide,

6. An X-ray contrast medium comprising a non-ionic contrast medium according to claim 1 in a physiologically acceptable carrier.

7. An X-ray contrast medium comprising a non-ionic contrast medium according to claim 5 in a physiologically acceptable carrier.

8. An X-ray contrast medium comprising a non-ionic contrast medium according to claim 6 in a physiologically acceptable carrier.

9. A method for the non-invasive determination of a physiological condition using irradiation and a non-ionic contrast medium, characterised in that a non-ionic contrast medium according to claim 1 is used.

10. A process for the preparation of compounds of the general formula I, characterised in that

a) compounds of the general formula V

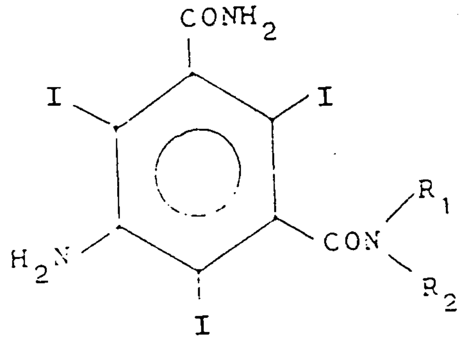

(V)

wherein hydroxy groups contained in $R_1$ and $R_2$ are optionally protected, are converted by reaction with $R_3CO$-X, wherein X represents halogen or an ester radical, in catalysing solvents such as, for example, pyridine, DMA or DMF, with subsequent removal of protecting groups where appropriate, into end products of the general formula I wherein $R_4$ represents hydrogen or into intermediates of the general formula I' wherein $R_4$ represents $R_3CO$, and those, optionally with protected hydroxy groups, are then if desired converted by alkylation under basic conditions with $R_4$-containing reagents, after subsequent removal of the protecting groups where appropriate, into end products of the general formula I in which $R_4$ does not represent hydrogen, or

b) compounds of the general formula VI

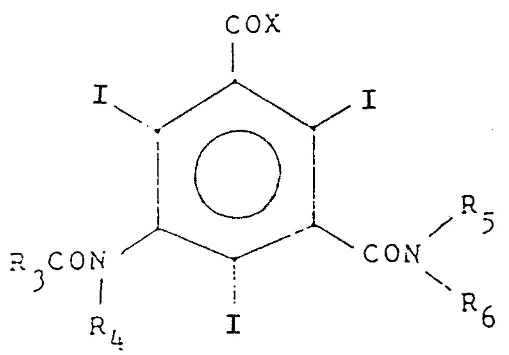

(VI)

wherein $R_5$ represents $R_1$ or hydrogen, $R_6$ represents $R_2$ or hydrogen and X represents halogen or an ester radical, are reacted with ammonia or with hydroxyalkylamines containing radicals $R_1$ and $R_2$ the hydroxy groups of which may be protected, and then the protecting groups are where appropriate removed.

## Revendications

**1.** Agent de contraste non ionique de formule (I) :

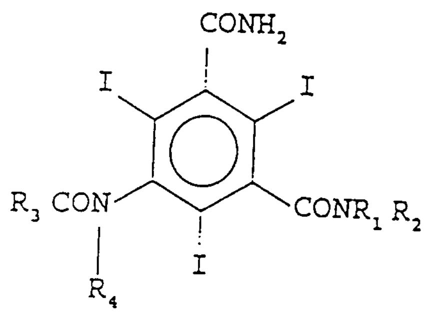

(I)

ayant au moins deux groupes hydroxy ;
dans laquelle

- $R_1$ signifie un hydrogène, un alkyle inférieur ou un alkyle inférieur hydroxy-substitué, le groupe alkyle possédant entre 1 et 6 atomes de carbone ;
- $R_2$ signifie un hydroxyalkyle ayant entre 2 et 6 atomes de carbone, lequel possède entre 1 et n - 1 groupes hydroxyle, n étant le nombre d'atomes de carbone ;
- $R_3$ signifie un alkyle inférieur, un alkyle inférieur hydroxy-substitué ou un alkyle inférieur alcoxy-substitué ayant de 1 à 6 atomes de carbone, ou un reste de formule II :

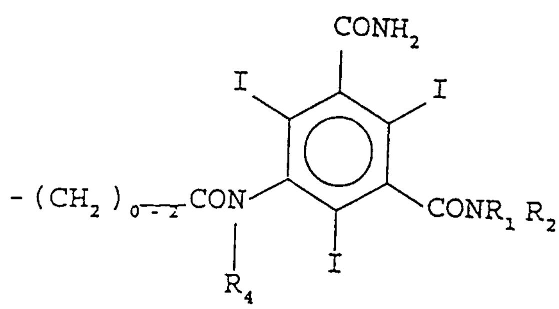

(II)

- $R_4$ signifie un hydrogène ou un alkyle ayant de 1 à 6 atomes de carbone, lequel possède de 0 à n - 1 groupes hydroxyle, n étant le nombre d'atomes de carbone,

à l'exception du 3-[N-(1,3-dihydroxyprop-2-yl)carbamoyl]-5-(2-hydroxy-1-oxopropyl)-amino-2,4,6-triiodo-benzamide.

**2.** Agent de contraste non ionique selon la revendication 1, caractérisé en ce que $R_2$ possède 4 atomes de carbone et 3 groupes hydroxyle, $R_3$ n'est pas représenté par un reste de formule II et $R_4$ possède entre 2 et 3 atomes de carbone et au moins un groupe hydroxyle.

**3.** Agent de contraste non ionique selon la revendication 1, caractérisé en ce que $R_2$ possède 3 atomes de carbone et 2 groupes hydroxyle, $R_3$ n'est pas représenté par un reste de formule II et $R_4$ possède 3 atomes de carbone et 2 groupes hydroxyle.

**4.** Agent de contraste non ionique selon la revendication 3, caractérisé en ce que $R_2$ et $R_4$ sont identiques.

**5.** 5-{N-(2-Hydroxyéthyl)-acétamido}-2,4,6-triiodo-3-{N-(1,3,4-trihydroxy-thréo-but-2-yl)}-carbamoyl-benzamide.

5-{N-(2,3-Dihydroxypropyl)-acétamido}-2,4,6-triiodo-3-{N-(2,3-dihydroxypropyl)}-carbamoyl-benzamide.

5-{N-(2,3-Dihydroxypropyl)-acétamido}-2,4,6-triiodo-3-{N-(2-hydroxyéthyl)}-carbamoyl-benzamide.

5-{N-(Méthyl)-2-hydroxyacétamido}-2,4,6-triiodo-3-{N-(1,3,4-trihydroxy-thréo-but-2-yl)}-carbamoyl-benzamide.

Acide bis-{3-[N-(1,3,4-trihydroxy)-thréo-but-2-yl}-carbamoyl-5-carbamoyl]-2,4,6-triiodo-N-(méthyl)-anilide-malonique.

Acide bis-[{3-N-(2,3-dihydroxypropyl-carbamoyl)-5-carbamoyl}-2,4,6-triiodo-N-(2,3-dihydroxypropyl)anilide-malonique.

Acide bis-[{3-N-(2,3-dihydroxypropyl-carbamoyl)-5-carbamoyl}-2,4,6-triiodo-N-(2-hydroxyéthyl)-anilide-malonique.

5-[N-(2-Hydroxyéthyl)-hydroxyacétamido]-2,4,6-triiodo-3-[N-(2,3-dihydroxypropyl)]-carbamoyl-benzamide.

3,5-[N,N'-(2,3-Dihydroxypropyl)-diacétamido]-2,4,6-triiodo-benzamide.

**6.** Agent radiologique consistant en un agent de contraste non ionique selon la revendication 1 dans un support compatible du point de vue physiologique.

**7.** Agent radiologique consistant en un agent de contraste non ionique selon la revendication 5 dans un support compatible du point de vue physiologique.

**8.** Agent radiologique consistant en un agent de, contraste non ionique selon la revendication 6 dans un support compatible du point de vue physiologique.

**9.** Procédé de détermination non invasif d'un état physiologique en utilisant des rayonnements et un agent de contraste non ionique, caractérisé en ce que l'on utilise un agent de contraste non ionique selon la revendication 1.

**10.** Procédé de préparation de composés de formule générale I, caractérisé en ce que

a) on transforme des composés de formule générale V :

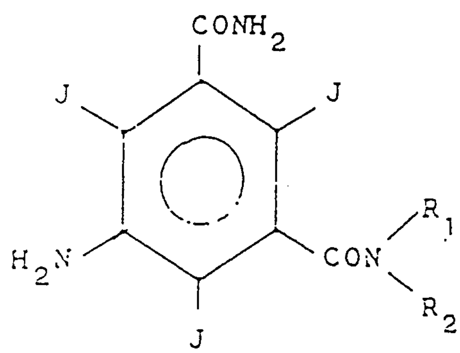

(V)

dans laquelle les groupements hydroxy contenus dans $R_1$ et $R_2$ sont éventuellement protégés, par réaction avec $R_3CO$-X, X représentant un halogène ou un reste ester, dans un solvant jouant un rôle de catalyseur comme par exemple la pyridine, le DMA ou le DMF et éventuellement par élimination ultérieure des groupements protecteurs conduisant respectivement aux produits finaux de formule générale I avec $R_4$ ayant la signification d'un hydrogène ou aux produits intermédiaires de formule générale I avec $R_4$ ayant la signification de $R_3CO$ et l'on transforme ceux-ci, avec des groupements hydroxy étant éventuellement protégés, ensuite dans le cas souhaité par alkylation sous condition basique avec des réactifs contenant $R_4$, éventuellement après élimination consécutive des groupements protecteurs, conduisant aux produits finaux de formule générale I, dans laquelle $R_4$ ne signifie pas un hydrogène, ou bien

b) on transforme des composés de formule générale VI :

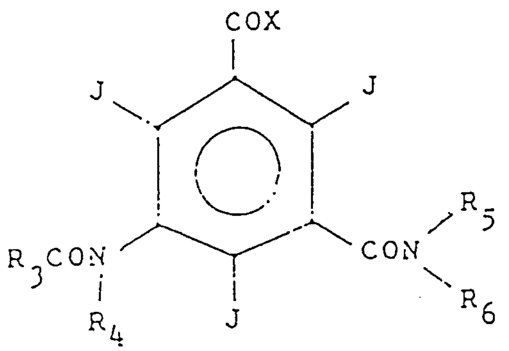

(VI)

avec $R_5$ ayant la signification de $R_1$ ou d'un hydrogène, $R_6$ ayant la signification de $R_2$ ou d'un hydrogène et X ayant la signification d'un halogène ou d'un reste ester,

avec de l'ammoniac (ou de l'ammoniaque) ou bien avec des hydroxyalkylamines contenus dans les restes $R_1$ et $R_2$, lesquels groupes hydroxy pouvant être protégés, et l'on élimine ensuite éventuellement les groupements protecteurs.